(19) Europäisches Patentamt
European Patent Office
Office européen des brevets

(11) EP 1 009 837 B1

(12) EUROPEAN PATENT SPECIFICATION

(45) Date of publication and mention of the grant of the patent:
**16.04.2003 Bulletin 2003/16**

(21) Application number: **98942845.3**

(22) Date of filing: **04.09.1998**

(51) Int Cl.[7]: **C12N 15/81**, C12P 7/06, C12N 1/18 // C12P7:06, C12R1:865

(86) International application number:
**PCT/GB98/02632**

(87) International publication number:
**WO 99/011804 (11.03.1999 Gazette 1999/10)**

(54) **ETHANOL PRODUCTION BY MUTANT YEAST**

ETHANOLHERSTELLUNG DURCH MUTANTHEFE

PRODUCTION D'ETHANOL A L'AIDE DE LEVURE MUTANTE

(84) Designated Contracting States:
**DE ES FR GB IE IT**

(30) Priority: **04.09.1997 GB 9718711**

(43) Date of publication of application:
**21.06.2000 Bulletin 2000/25**

(73) Proprietor: **Sachet-Pack Limited**
**Prenton, Wirral L43 3DU (GB)**

(72) Inventors:
- **OLIVER, Stephen George**
  **Stockport, Cheshire SK12 2DW (GB)**
- **HUTTER, Anton**
  **Chorlton, Manchester M21 8AR (GB)**

(74) Representative: **Brierley, Anthony Paul et al**
**Appleyard Lees**
**15 Clare Road**
**Halifax West Yorkshire HX1 2HY (GB)**

(56) References cited:
**WO-A-98/41640** **US-A- 4 567 145**

- **BROWN S.W. ET AL.: "Ethanol production and tolerance in grande and petite yeasts." JOURNAL OF CHEMICAL TECHNOLOGY AND BIOTECHNOLOGY, PART B - BIOTECHNOLOGY., vol. 34B, no. 2, 1984, pages 116-120, XP002070575 OXFORD GB**
- **GLERUM D.M. & TZAGOLOFF A.: "Submitochondrial distributions and stabilities of subunits 4, 5, and 6 of yeast cytochrome oxidase in assembly defective mutants." FEBS LETTERS., vol. 412, no. 3, 4 August 1997, pages 410-414, XP002070576 AMSTERDAM NL**
- **MCEWEN J.E. ET AL.: "Sequence and chromosomal localisation of two PET genes required for cytochrome c oxidase assembly in Saccharomyces cerevisiae." CURRENT GENETICS, vol. 23, no. 1, January 1993, pages 9-14, XP002070577**
- **AGUILERA A. & TAHIA B.: "Role of mitochondria in ethanol tolerance of Saccharomyces cerevisiae." ARCHIVES IN MICROBIOLOGY, vol. 142, no. 4, - 1985 pages 389-392, XP002070578**
- **HUTTER A. & OLIVER S.G.: "Ethanol production using nuclear petite yeast mutants." APPLIED MICROBIOLOGY AND BIOTECHNOLOGY, vol. 49, no. 5, May 1998, pages 511-516, XP002070579**
- **BAGANZ F ET AL: "Quantitative analysis of yeast gene function using competition experiments in continuous culture." YEAST, vol. 14, no. 15, November 1998, pages 1417-1427, XP002092982**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 1 009 837 B1

## Description

The present invention relates to the production of ethanol using a specific type of yeast cell.

Ethanol is widely produced by a fermentation process in which yeast converts carbohydrates into ethanol.

There are several factors which limit the yield of ethanol from such fermentations. For instance, oxygen can have a deleterious effect on the yield of ethanol (known as the Pasteur effect) and under aerobic conditions ethanol tends to be lost by further metabolism (primarily by mitochondrial respiration). Another factor influencing ethanol production is the amount of carbohydrate in the fermentation. The provision of excess carbohydrate tends to inhibit respiration in certain strains of yeast (known as the Crabtree effect) and thereby allows ethanol production even in the presence of oxygen. Therefore the yield of ethanol from Crabtree-effect-sensitive yeast may be maximized by increasing the concentration of carbohydrate and alternatively or additionally the yield of ethanol may be maximized from any yeast by restricting oxygen availability. However, despite such measures, actual yields of ethanol are less than is desirable. A further factor which limits ethanol yield relates to the fact that yeast are generally intolerant of high ethanol concentrations. As the concentration of ethanol increases the growth rate of yeast cells is first retarded and then as the concentration increases further cell viability is adversely effected.

Various attempts have been made to provide yeast cells which produce high yields of ethanol. One such attempt has involved the development of yeast mutants which are respiratory deficient because they contain non-functional mitochondrial genes (also known as mitochondrial or cytoplasmic petite mutants). Such mutants are not subject to the Pasteur effect and therefore strict control of the oxygen supply to the fermentation was not essential, as is the case for fermentations employing respiratory sufficient (grande) strains of yeast. BROWN S.W. ET AL.: 'Ethanol production and tolerance in grande and petite yeasts.' JOURNAL OF CHEMICAL TECHNOLOGY AND BIOTECHNOLOGY, PART B - BIOTECHNOLOGY., vol. 34B, no. 2, 1984, pages 116-120, OXFORD, GB (**D1**) discloses e.g. the production of ethanol by cytoplasmic petite mutant.

However, when oxygen in the feed gas was restricted, the specific rate of ethanol production was found to be higher in the parental grande strain ($\rho^+$) than in the petite mutant, provided that the supply of oxygen to the $\rho^+$ culture was maintained at a level sufficient for sterol and unsaturated fatty acid biosynthesis.

It is therefore an object of the present invention to obviate or mitigate the above mentioned disadvantages.

According to a first aspect of the present invention there is provided a method of producing ethanol comprising growing respiratory deficient yeast cells which have at least one nuclear gene, or product thereof, required for respiration which is non-functional and/or inhibited in a growth medium and separating ethanol from said yeast cells.

By "a respiratory deficient yeast cell" we mean a yeast cell with no mitochondrial mediated respiration (i.e. 100% respiratory deficient) or a yeast cell with a reduction in respiration of at least 60%, preferably at least 75%, more preferably 85%, or even more preferably 95% relative to a corresponding yeast cell in which the at least one nuclear gene, or product thereof, required for respiration is functional and/or not inhibited. For instance, a nuclear gene of a parental grande strain ($\rho^+$) may be mutated or a gene product inhibited such that mitochondrial respiration is reduced by at least 60% relative to the unmanipulated parental grande strain.

The inventors have found that yeast cells in which nuclear genes that promote, or are required for, mitochondrial respiration are non-functional (or when the gene product thereof is inhibited) are insensitive or less sensitive to the Pasteur effect and have ethanol tolerance that is comparable to unmanipulated cells and better than mitochondrial petite variants. Accordingly, yeast cells used according to the method of the invention offer great benefits when used in the production of ethanol because it is not necessary to maintain the cells in strict anaerobic conditions and the yield of ethanol from each batch of cultured yeast cells is able to reach higher concentrations than is possible with previously known respiratory deficient yeast strains. The cells are of particular use in the commercial production of ethanol because the oxygen supply need not be tightly controlled. Furthermore, the cells are not able to grow in a diauxic growth phase and so will not metabolize the product of fermentation (i.e. ethanol) as their secondary substrate. This allows higher final titres to be achieved and also obviates the necessity for quickly arresting a culture of yeast (for fear of losing yield).

While we do not wish to be bound by any hypothesis, we believe that respiration deficient yeast cells used according to the method of the invention (also referred to herein as nuclear petite yeast mutants) are capable of yielding high amounts of ethanol because they are less sensitive to ethanol than mitochondrial petite mutants. We have established that exogenously added ethanol retards the growth of mitochondrial petite mutants compared to that of the grande parent yeast cells whereas nuclear petite mutant yeasts exhibit better growth rates in the presence of ethanol than mitochondrial petites. Furthermore, we have established that nuclear petite mutants produce ethanol at a faster rate than mitochondrial petites or the grande parents ($\rho^+$) from which the nuclear petites are derived. In mitochondrial petite mutants, we believe that the lack of an intact mitochondrial genome causes several deleterious cell surface characteristics (such as reduced sugar uptake, altered cell flocculation and agglutination and an effect on plasma membrane proteins) that results in a decrease in their ethanol tolerance. Mitochondria exist in two different, well-defined physiological states depending on the presence or absence of dissolved oxygen (a promitochondrial form and a functional mitochondrial form). The promitochondrial form is produced during anaerobic growth and differentiates into a fully functional mitochondrion when minute quantities of dissolved oxygen are made available. Over recent years, mitochondrial function in brewers' yeast has been largely ignored due to the Crabtree effect, i.e. yeast respiratory activity is repressed by glucose under aerobic conditions. Since brewing yeasts are Crabtree-positive, the role of oxygen is considered to be solely nutritional and the existence of any true respiratory functions in brewing yeast is usually denied. The transformation of promitochondria into mitochondria is a metabolically expensive undertaking yet this phenomenon has always been observed in brewing yeasts but its significance never explained. We therefore propose that fully developed functional mitochondria are, in an as yet unknown manner, essential for maintaining the ethanol tolerance and, consequently, fermentation performance of yeast. This explains the ineffectiveness of the mitochondrial petite yeast as a means of producing ethanol and explains why nuclear petite yeasts are surprisingly so effective.

Respiratory deficient yeast cells may be derived from any yeast strain. However it is preferred that the cells are derived from Brewer's yeast such as *Saccharomyces cerevisiae*. Other yeasts which may be used include *S. uvarum (S. carlsbergensis)* and other members of the *Saccharomyces sensu stricto* group.

A preferred yeast strain from which the respiratory deficient yeast strain may be derived is FY23 (Winston *et al*. (1995) Yeast 11:p53-55) and related strains. Most preferred derivatives of FY23 for use according to the method of the invention are those discussed in detail below and in Example 1, namely FY23Δ*cox5a* and FY23Δ*pet191*. We have found that FY23Δ*pet191* is 100% respiratory deficient (or at least substantially so) and represents a particularly useful yeast strain for use according to the method of the first aspect of the invention.

It is most preferred that yeast strains from which the respiratory deficient yeast strain may be derived are those strains which are already known to the art as good producers of ethanol. For instance, industrial high alcohol producing yeasts such as M-strains (DC(Y)L, Menstrie, Scotland), Red Star (Fleischmann's USA), K1 Yeast (Lallamand U.K. LTD, Fife, Scotland and produced by Danstar Ferment AG), Oenological Yeast U.C.L.M. S325 (Bio Springer, Maisins-Alfort, France) and Oenological Yeast U.C.L.M. S377 (Bio Springer, Maisins-Alfort, France) may be manipulated to develop respiratory deficient yeasts for use according to the method of the invention. A most preferred industrial yeast strain from which respiratory deficient yeast strains may be derived is K1. Most preferred derivatives of K1 for use according to the method of the invention are those discussed in detail below and particularly K1Δ*pet191ab* described in Example 2.

For examples of other suitable yeasts, which may be genetically manipulated for use in the method of the invention, see: "Yeast breeding for fuel alcohol production" by Tavares F.C.A., Echeverriagary (p59-79) in "Research on industrial yeasts vol. 1" (1987) Eds. Stewart G.G., Russel I.. Klein R.D., Hiebsch R.R. CRC Press.

A preferred means of generating yeast cells for use according to the method of the invention is to modify a nuclear gene that is required for respiration such that the gene is non-functional (i.e. to functionally delete the nuclear gene). This modification may be to cause a mutation, which disrupts the expression or function of the gene product. Such mutations may be to the nucleic acid sequences that act as 5' or 3' regulatory sequences for the gene or may preferably be a mutation introduced into the coding sequence of the gene. Functional deletion of the gene may, for example, be by mutation of the gene in the form of nucleotide substitution, addition or, preferably, nucleotide deletion.

The gene may be made non-functional by:

(i) shifting the reading frame of the coding sequence of the gene;
(ii) adding, substituting or deleting amino acids in the protein encoded by the gene; or
(iii) partially or entirely deleting the DNA coding for the gene and/or the upstream and downstream regulatory sequences associated with the gene.

A preferred means of introducing a mutation into a chromosomal gene required for respiration is to utilize molecular biology techniques specifically to target the chromosomal gene which is to be mutated. Mutations may be induced using a DNA molecule. A most preferred means of introducing a mutation is to use a DNA molecule that has been specially prepared such that homologous recombination occurs between the target gene and the DNA molecule. When this is the case, the DNA molecule will ideally contain base sequences complementary to the target chromosomal location to allow the DNA molecule to hybridize to (and subsequently recombine with) the target. It is particularly preferred that respiratory deficient nuclear petite mutants are generated using a PCR-mediated gene replacement technique such as the methods developed by Wach *et al*. (1994) Yeast 10:1793-7876.

Alternative means of effecting gene disruption to generate nuclear petite yeasts are described in Methods in Enzymology (1983) Vol 101 Recombinant DNA Part C (Eds Wu R., Grossman L., Moldave K.) see pages 202-228.

Nuclear genes which may be modified are those which, when made non-functional, give rise to the nuclear petite phenotype. These genes are usually associated with the function or expression of proteins involved either directly or indirectly in oxidative phosphorylation.

Preferred genes which may be made non-functional and/or inhibited are associated with the function of cytochrome c oxidase (the final electron carrier complex in oxidative phosphorylation).

Introducing a mutation into the PET191 gene produces a preferred yeast cell in which the function of cytochrome c oxidase is attenuated. *PET191* is found on yeast chromosome X and encodes a 14.1kDa protein required for the assembly of the polypeptide subunits which constitute the active cytochrome c oxidase holoenzyme. Sequence data for the *PET191* gene and adjacent DNA is available at:
http://genome.www.stanford.edu:8000/acedb/SacchDB?find+DNA+%22cseqX_16+%28 SGD%29%22).

A preferred mutation of the *PET191* gene produces a nuclear petite mutant that causes failure of the holoenzyme assembly and induces up to 100% respiratory deficiency. A most preferred cell with a mutation of *PET191* is the nuclear petite mutant named herein as FY23Δ*pet191* in which the DNA sequence identified as SEQ ID NO. I has been excised from yeast genome chromosome X and replaced with the DNA sequence identified as SEQ ID NO. 2.

Another most preferred cell with a mutation of *PET191* is the nuclear petite mutant named herein as K1Δ*pet191 ab*. K1WT is diploid and K1Δ*pet191 ab* has one copy of the *PET191* gene (*PET191a*) in which the DNA sequence identified as SEQ ID NO. 1 has been excised and replaced with the DNA sequence identified as SEQ ID NO. 17. The second copy of *PET191* (*PET191b*) in K1Δ*pet191 ab* has the DNA sequence identified as SEQ ID NO. 4 excised and replaced with the DNA sequence identified as SEQ ID NO. 17.

Cells with mutations of the *PET191* gene represent an important feature of the invention and according to the second aspect of the present invention there is provided a yeast cell characterized in that the *PET191* gene is functionally deleted.

Preferably yeast cells according to the second aspect of the invention are derived from wild type yeast strains which are respiratory sufficient in which the *PET191* gene is functionally deleted by homologous recombination between a DNA molecule and the DNA encoding the *PET191* gene.

Preferred yeast cells according to the second aspect of the invention are FY23Δ*pet*191 and K1Δ*pet191ab* as characterized herein.

Introducing a mutation into the COX5a gene located on chromosome XIV produces another preferred yeast cell for use in the method of the invention in which the function of cytochrome c oxidase is attenuated. *COX5a* codes for subunit V of Cytochrome c oxidase consists of nine polypeptide subunits. Sequence data for the *COX5a* gene and adjacent DNA is available at:
http://genome.www.stanford.edu:80001acedb/SacchDB?find+DNA+%22YSCCOX5AA+ %28GB%29%22.

A most preferred cell with a mutation of the *COX5a* gene is the nuclear petite mutant named herein as FY23Δ*cox5a* in which the DNA sequence identified as SEQ ID NO. 5 has been excised from the yeast genome (chromosome XIV) and replaced with the DNA sequence identified as SEQ ID NO. 2 Preferably the FY23Δ*cox5a* mutant is 85-90% respiratory deficient

Other nuclear genes that when made non-functional and/or inhibited in a yeast to give rise to nuclear petite yeasts, may be used according to the method of the invention may be selected from the group of yeast genes known as *PET* genes (e.g. *PET* 192 or *PET* 100) Examples of these *PET* genes are publicly available on the yeast genome database and are also described in Tzagaloff A. & Dieckman C.L. (1990) "PET genes of *S. cerevisiae*" Microbiol Rev. (54) 9:211-225. Other examples of nuclear genes that may be manipulated to form nuclear petite yeast cells include other *COX* genes (e.g. any one of *COX* 1 - 17), *ATP* genes, *CEM* 1, *CYB*2 and *QCR* genes (e.g. any one of *QCR* 1 - 10).

Preferred nuclear petite yeasts for use according to the method of the invention have nuclear genes which when made non-functional and/or inhibited give rise to a phenotype which is at least 95% respiratory deficient and most preferably 100% respiratory deficient. In this respect we have found that functional deletion of *PET* genes and particularly *PET 191* can result in yeasts at least 95% respiratory deficient.

When yeast cells according to the second aspect of the invention or yeast cells used according to the method of the invention are formed by the induction of a mutation in a parental wild type strain with a DNA molecule, the DNA molecule used to transform the parental yeast strain may be contained within a suitable vector to form a recombinant vector. The vector may, for example, be a plasmid, cosmid or phage. Such recombinant vectors are of great utility when replicating the DNA molecule. A preferred vector is pFA6-kanMX4 (see SEQ ID NO. 6) Another preferred vector is pUG6 (disclosed in Guldener *et al*. (Nucl. Acids. Res. (24) 13:2519-2524, 1996) that comprises DNA corresponding to pFA6-kanMX4 except the kanMX4 gene is flanked with LoxP sequences (discussed below and containing 34 bp LoxP sequences of SEQ ID. NO. 17).

The recombinant vectors and /or the DNA molecule will frequently include one or more selectable markers to enable selection of cells transfected with the vector and/or the nuclear petite mutant. An example of such a selectable marker is the inclusion of a gene, which confers resistance to geneticin (G418) such as KanMX4. For instance a preferred way of producing a cell which may be used in the method of the invention is to transform a respiratory sufficient parental strain of yeast with a DNA cassette comprising DNA coding a marker gene flanked by DNA which will allow homologous recombination with a target nuclear gene encoding a protein involved in respiration.

When selectable markers are used it is often desirable to excise the marker from the genome after the genetic manipulation has been effected (as is the case for K1Δ*pet191ab* - see Example 2). When this is the case various known recombinase enzyme systems may be used to excise the marker. These systems exploit the ability of recombinases to cleave specific nucleotide repeat sequences. The nucleotide repeat sequences may be included in the DNA cassette used to transform the yeast cell such that they flank the DNA encoding the marker. Following transformation of the yeast to form a nuclear petite, the nucleotide repeat sequences may be excised by use of the recombinase. An example of a system which may be used to excise a marker gene is the LoxP/Cre system described in more detail in Example 2 for K1Δ*pet191ab*.

An alternative to the loxP/Cre recombinase system for the *in vivo* excision of the kanMX module would be to use the meganuclease, I-*Sce*I. The restriction site of I-*Sce*I is encoded by a mobile group I intron of yeast mitochondria and is 18bp long and deletion cassettes may be designed comprising the kanMX module flanked by an I-*Sce*I site either side (instead of the loxP sites). Additionally, the outer flanking regions of these I-SceI sites should be homologous to one another such that, upon excision of the kanMX module by expression of the endonuclease I-Sce1, the linearised genomic DNA would religate, repairing the DNA. An advantage of this technique would be the absence of any heterologous DNA left in the deleted strain.

Thus most preferred nuclear petites according to the second aspect of the invention or for use according to the method of the invention are formed by transforming respiratory sufficient yeasts with DNA cassettes comprising DNA coding a marker gene flanked by DNA which will homologously recombine with a target nuclear gene encoding a protein involved in respiration and further comprising the nucleotide repeat sequences discussed in the above paragraph.

It is also possible to provide a respiratory deficient yeast cell for which at least one chromosomal gene product required for respiration is non-functional and/or inhibited without introducing a mutation into the yeast genome. This may be done by using specific inhibitors that will prevent respiration occurring in yeast. Examples of such inhibitors include agents that prevent transcription of the gene, prevent expression or disrupt post-translational modification. Alternatively, the inhibitor may be an agent that increases degradation of the gene product (e.g. a specific proteolytic enzyme). Equally the inhibitor may be an agent which prevents the gene product from combining with mitochondrial components such as a neutralizing antibodies (for instance an anti-*PET191* antibody). The inhibitor may also be an antisense oligonucleotide or any synthetic chemical capable of inhibiting components of respiration that are mediated by nuclear genes.

The methods of the invention are of great benefit because the rate of ethanol production and, in certain circumstances, the yield of ethanol produced is higher than for known methods that utilize conventional yeast strains. The methods may be used for the production of ethanol for any purpose (including potage) although the methods are particularly suited for the commercial production of ethanol for the chemical industry and most particularly for the production of fuel ethanol.

The growth medium may be any conventionally used growth medium for yeast (for instance, YPD growth medium) and should ideally contain a source of carbohydrate, which may be a fermentable sugar. For instance, the carbohydrate may be a derivative of sugar beet or sugar cane (such as cane juice or molasses). Preferred carbohydrates for metabolic conversion into ethanol are sucrose or glucose.

The growth medium should contain ideal amounts of required nutrients (i.e. carbohydrate, nitrogen source etc) to allow optimal growth and ethanol production. The exact composition of the medium depends upon a number of factors (for instance the specific yeast used). Purely by way of example a suitable growth medium may comprise:

| | |
|---|---|
| Urea | 2.34 g/L |
| Mono-Potassium Phosphate | 1.49 g/L |
| Magnesium Sulphate | 0.9 g/L |
| Inositol | 0.1 g/L |
| Nicotinic Acid | 0.07 g/L |
| Pyridoxine Hydrochloride | 0.005 g/L |
| Thiamine Hydrochloride | 0.008 g/L |
| Calcium Pantothenate | 0.005 g/L |
| Biotin | 0.0002 g/L |
| Zinc Sulphate | 0.006 g/L |
| Di-Ammonium Iron (II) Sulphate | 0.004 g/L |
| Copper Sulphate | 0.0001 g/L |
| + carbohydrate substrate | |

The concentration of carbohydrate substrate influences the amount of ethanol produced and, generally, the higher the concentration of carbohydrate. the greater the yield of ethanol. However, if the concentration of carbohydrate is too high, it can induce plasmolysis in yeast due to osmotic effects. The extent of plasmolysis is in turn dependent upon the fermentation conditions and the type of yeast used. The exact amount of carbohydrate added to a fermentation must therefore be tailored to the exact requirements of a given yeast culture. By way of example addition of 351 g/L of sucrose to the abovedescribed media can achieve optimal v/v ethanol production under suitable fermentation conditions:

Media may also be supplemented with various other nutrients such as complex nitrogenous sources (e.g. peptides, amino acids), other vitamins or fatty acids and other lipids.

Media should also be buffered such that the pH is maintained between pH 4 and 7 and most preferably between pH 4.5 and 5 during the fermentation process. The temperature of the medium during fermentations may be maintained at ambient temperatures (in the region of 20 °C) for optimal ethanol production although some mesophilic and thermophilic strains of yeast have optimal temperature ranges for growth in the region of 28-35 °C and 40-50 °C respectively.

Optimal yeast growth and ethanol production is at least partially dependent upon how the yeast is stored before use and then how it is subsequently introduced into the growth medium. The yeast may be supplied in a dried form and then rehydrated before pitching into the fermentation media. Yeast is best rehydrated in 35-40 °C water (10ml/g of yeast) for 15 minutes. The optimal amount of dried yeast added to fermentation media is in the region of 2.13 g/L of active yeast, assuming approximately 1 x$10^{10}$ colony forming units per gramme.

The yeast is grown in a fermenter containing the growth medium. This may be any vessel in which yeast cells can be grown. Fermenters for production of ethanol on an industrial scale (for instance production of fuel ethanol) will usually further comprise one or more of:

1. Rotors or similar devices for agitating the yeast culture.
2. An air (or oxygen) supply.
3. An inlet for addition of nutrients.
4. A means of extracting waste products and / or ethanol produced.
5. A thermostat and means of regulating temperature.

Preferred fermenters are those already known to the art for the culture of yeast. The type of fermenter used will depend upon whether the yeast cells are being grown in the laboratory, by potage, as a pilot plant or in full scale-up for industrial production of ethanol. For instance, when the yeast cells are being used for experimental purposes, a preferred fermenter is the 3L Bioengineering LKF2000 basic fermenter (Bioengineering AC, CH8636-Wald, Switzerland).

The fermenters may have means for automating the regulation of temperature, air supply, nutrient content, waste removal and product extraction. A preferred means of automation is the use of a "Pi" computer control system (Process Intelligence Ltd., Warwick, UK) to electronically control the fermentation.

The methods for producing ethanol may involve the growth of yeast cells by batch or continuous culture.

When ethanol is produced for certain reasons (e.g. industrial production of ethanol) the method requires the step of separating ethanol from said yeast cells. The ethanol may be separated from the yeast cells by any convenient method. For the industrial production of ethanol (e.g. fuel ethanol) this may involve several steps including a distillation step and/or a filtration step. If the cells are used for potage the ethanol may be isolated from the cells by means of settling, flotation or filtering.

It is desirable to leave the ethanol in the medium in which it is grown in the brewing of some alcoholic drinks are brewed (especially drinks containing about 20% ethanol or less e.g. beers). When this is the case, rather than the ethanol being separated it is often desirable to separate the yeast from the medium instead (e.g. by sedimentation).

In a specific embodiment of the method of the invention yeast cells which have at least one nuclear gene, or product thereof, required for respiration which is non-functional and/or inhibited (e.g. K1Δ*pet191 ab* or FY23Δ*pet191*) are grown in a complete YPD medium [1% (w/v) yeast extract, 2% (w/v) peptone, 5% (w/v) glucose] within 3L Bioengineering LKF2000 basic fermenters as a batch culture. After 45 hours culture time the medium containing ethanol is removed and subsequently used or then treated to isolate the ethanol. Monitoring and control of fermentations are achieved using a "Pi" computer control system. Growing cultures are agitated at 850 r.p.m. by using 2 x 6-blade Rushton impellers mounted on a central shaft and driven by a 250W VDE electric motor (Lenze, D-4923, Germany). Excessive foaming is prevented by the addition of several drops of a silicon-based anti-foaming agent (Mazu DF8005). Cultures are aerated at 1Lmin$^{-1}$ (1v/v/m) using a rotameter and precision control valve. Fermenters are equipped with a 405-DPAS-50-K85/200 combination pH electrode and the pH of all fermentations maintained at 5.0 by the addition of 3M HCl and 3M NaOH solutions via W/M 501u pumps (Watson and Marlow, UK). Control of pH is implemented, via the "Pi" system, using an on/off control loop about the set point of pH 5.0 (±0.005). All fermentations are carried out at 30°C (±0.005°C) using the proportional (P) and Integral (I) controller of the "Pi" system. Dissolved oxygen in the medium is continuously monitored by means of a DO probe (Ingold, UK), as is $CO_2$ in the exhaust gas using an infrared gas analyser (ADC Instruments Ltd., Hoddesden, UK).

It will be appreciated that the abovedescribed embodiment of the method of the invention may be readily adapted using conventional biochemical engineering techniques for the scale-up of ethanol production. Guidance for the scale-up of ethanol production using conventional yeast (which may be readily adapted for use with nuclear petite yeasts) can be found in "Research on industrial yeasts vol. 1" (1987) Eds. Stewart G.G., Russel I., Klein R.D. & Heibsch R.R. CRC Press or "Biochemical Engineering & Biotechnology Handbook" (1991) Atkinson B. & Mavituna F. The Nature Press. Examples of known means by which scale up of ethanol production may be achieved are by using the Berkeley process, MIT process, Natick process or sugar-cane substrate method.

The present invention will now be described, by way of example, with reference to the accompanying drawings in which:

Figure 1 is a schematic representation of the methods used to generate respiratory deficient yeast cells in Example 1;
Figure 2 is a schematic representation of DNA coding for the *PET191* open reading frame and adjacent nucleotides showing where the relevant PCR primers used in Example 1 bind to the DNA;
Figure 3 is a schematic representation of DNA coding for the *COX5a* open reading frame and adjacent nucleotides showing where the relevant PCR primers used in Example 1 bind to the DNA;
Figure 4 is a graph illustrating fermentation data for yeast strain FY23WT of Example 1;
Figure 5 is a graph illustrating fermentation data for yeast strain FY23Δ*pet*191 of Example 1;
Figure 6 is a graph illustrating fermentation data for yeast strain FY23Δ*cox5a* of Example 1;
Figure 7 is a graph illustrating fermentation data for yeast strain FY23 [ρ°] of Example 1;
Figure 8 is a schematic representation of the vector pUG6 used to generate the deletion cassette in Example 2;
Figure 9 is a schematic representation of the methods used to generate respiratory deficient yeast cells, K1Δ*pet191ab*, in Example 2;
Figure 10 is a schematic representation of the vectors. YEP351(a); YEP351-cyh(b) and YEP351-cre-cyh(c) used in Example 2; and
Figure 11 is a schematic representation of (I) the integrated deletion cassette in *PET 191a* and (2) the integrated deletion cassette in *PET 191b* of the respiratory deficient yeast cells in Example 2.

## EXAMPLE 1

Two types of nuclear petite yeast cells were generated from *Saccharomyces cerevisiae* strain FY23. Each cell was a nuclear petite having a mutation associated with the abolition or a reduction in cytochrome c oxidase activity. These yeast cells were then used according to the method of the invention to produce ethanol. The yeast cells had good tolerance to ethanol, circumvented the Pasteur effect by means of the nuclear lesion in the respiratory chain and achieved fermentation rates higher than that of the respiratory competent parental yeast (FY23WT) or of a mitochondrial petite derived from FY23.

### 1.1 METHODS

### 1.1.1 Yeast strains used

#### 1.1.1.1 FY23WT

*Saccharomyces cerevisiae* FY23 parental - haploid, grande [$\rho^+$], *MAT**a***, *ura*3-52, *trp*Δ63, *leu*2Δ1 (Winston *et al*., 1995, Yeast **11**:53-55) was used in these studies as the respiratory competent parental yeast. FY23WT was used as a control yeast strain in the fermentation studies and as the yeast strain from which respiratory deficient yeast cells were generated.

#### 1.1.1.2. FY23 ($\rho^0$)

The mitochondrial petite mutant FY23 ($\rho^0$) lacking all detectable mitochondrial DNA, as determined by equilibrium centrifugation in CsC1 Hoechst 33258 density gradients (Williamson *et al*., 1976. Methods in Cell. Biol. **12**: 335-351), was used in the fermentation studies for comparison with yeast cells according to the first aspect of the invention. The mitochondrial petite was derived from the grande parent by growth on YPD in the presence of 100µg/ml ethidium bromide. Cell growth on YPD but not on YEPG replica plates was indicative of petites (Slonimski *et a l*., 1968, Biochem. Biophys. Res. Comm. **30**:232-239).

**1.1.1.3 FY23Δ*pet*191**

FY23Δ*pet191* is a yeast cell according to the second aspect of the invention and may be used according to the method of the first aspect of the invention. FY23Δ*pet191* is a nuclear petite mutant of FY23 with disruption of the *PET191* gene. This mutation causes failure of cytochrome c oxidase holoenzyme assembly and thereby induces 100% respiratory deficiency. Details concerning the production of the mutant are given below.

**1.1.1.4. FY23Δ*cox5a*.**

FY23Δ*cox5a* is a yeast cell that may be used according to the method of the first aspect of the invention. FY23Δ*cox5a* is a nuclear petite mutant of FY23 with disruption of the *COX5a* gene and is approximately 85-90% respiratory deficient. Details concerning the production of the mutant are given below.

**1.1.2 Generation of nuclear petite mutants**

The respiratory deficient nuclear petite mutants were generated using the PCR-mediated gene disruption technique developed by Wach *et al*. (1994) Yeast **10**:1793-7876 and as outlined in Fig1 under 1) PCR.

A standard 50μl PCR reaction mix and conditions were used. Standard 50μl PCR reaction mix - 0.1mM dNTPs, 0.2μM primers. 1.5mM $MgCl_2$, 20ng template pFA6-kanMX4 (SEQ ID NO. 6), 2.5U *Taq* Polymerase. Standard PCR conditions - 1 cycle: 92°C for 2 min; 30 cycles: 92°C for 30s; 55°C for 30s; 72°C for 90s followed by 1 cycle: 72°C for 4 min.

**1.1.2.1. FY23Δ*pet191***

PCR was used to construct disruption cassette *pet191*::kanMX4 (1.3kb) (see Fig. 1(1)) in which a specific region of pFA6-MX4 was amplified using primers PET191 up (SEQ ID NO. 7) and PET191 down (SEQ ID NO. 8) (see Table 1). These primers having 19-22nt homology to the pFA6-MX4 multicloning site and 35nt extensions which were homologous, respectively, to the region immediately downstream of the start codon or to that upstream of the stop codon of the target ORF *PET191* (see Fig. 2).

Following correct amplification of the disruption cassette, 1-2μg of the ethanol-precipitated DNA was used for yeast transformation (Gietz *et al*., 1995). Upon transformation, recombination events took place between the homologous regions of the *pet191*::kanMX4 cassette and the target gene, thus disrupting the ORF and resulting in the excision of a DNA fragment with the sequence of SEQ ID NO. 1 and replacement with DNA encoding the KanMx4 gene with the sequence of SEQ ID NO. 2. Transformant selection took place on YEPD-agar containing 0.2mg/ml geneticin (G418).

**1.1.2.2 FY23Δ*cox5a***

Generation of a 85% respiratory-deficient FY23Δ*cox5a* was achieved as for FY23Δ*pet191* using the disruption cassette *cox5a*::kanMX4 which was the same as *pet191*::kanMX4 except the primers used were Cox5a Up (SEQ ID NO.11) and Cox5a Down (SEQ ID NO. 12) (from Table 1). These primers introduced DNA with homology to the *Cox5a* gene 5' and 3' of DNA encoding the KanMX4 gene in the cassette.

Transformation and recombination events took place as described in 1.1.2.1 to disrupt the ORF of *Cox5a* and resulting in the excision of a DNA fragment with the sequence of SEQ ID NO. 5 and replacement with DNA encoding the KanMx4 gene with the sequence of SEQ ID NO. 2. Transformant selection took place on YEPD-agar containing 0.2mg/ml geneticin (G418).

**1.1.3 Analytical PCR**

Correct targeting of each of the 2 disruption cassettes into the yeast genome was verified by analytical PCR (Wach *et al*., 1994). This was performed on the whole cell genomic DNA of transformants growing on 0.5mg/ml G418. Analytical PCR conditions - 1 cycle: 92°C for 2 min; 30 cycles: 92°C for 30s (for *PET191*) or 54°C for 30s (for *COX5a*); 72°C for 90s (*PET191*) or 72°C for 3 min (*COX5a*); followed by 1 cycle at 72°C for 4 min. Primers PET191 Forward and PET191 Reverse (see Table 1) were used in the analytical PCR reaction for FY23Δ*pet191* and primers Cox5a Forward and Cox5a Reverse (see Table 1) were used per PCR reaction for FY23Δ*cox5a* analytical PCR.

Further confirmation of correct ORF disruption was achieved by testing for respiratory deficiency on YEPG plates. The putative nuclear petites were also subjected to pulsed-field gel electrophoresis using the Contour-clamped Homogenous Electric Field (CHEF) technique in order to separate the chromosomes. This was followed by Southern

hybridisation whereby a $^{32}P$-labeled KanMX4 cassette was used to probe the membrane. Subsequent exposure to a phosphoimager (Biorad, UK) indicated that the *cox5a*::KanMX4 and *pet191*::KanMX4 cassettes had integrated correctly into chromosomes XIV and X respectively.

**TABLE 1. Oligonucleotide Primers used in PCR reactions of Example 1:**

| Oligonucleotide Primer DNA SEQ. ID No. | DNA sequence | Description |
|---|---|---|
| 7 | 5' - ATG TAA AGA TCA GAA GAA GGC TGT CGC TAT ATG TTC AGC TGA AGC TTC GTA CGC - 3' | **PET191 Up** (For construction of KanMX deletion cassette) |
| 8 | 5' - GAT TAC TGG ACA AAC CGA ATA AAA GAG CTT GAC GCC ATA GGC CAC TAG TGG ATC TG - 3' | **PET191 Down** (For construction of KanMX deletion cassette) |
| 9 | 5' - GCA CCT CTG TCG ACT GG - 3' | **PET191 Forward** (For checking correct chromosomal integration) |
| 10 | 5' - GAA AGC ACG TTA ACT CCC A - 3' | **PET191 Reverse** (For checking correct chromosomal integration) |
| 11 | 5' - CAC TTT TAC TAG AGC TGG TGG ACT ATC ACG TAT TAC AGC TGA AGC TTC GTA CGC - 3' | **Cox5a Up** (For construction of KanMX deletion cassette) |
| 12 | 5' - TCA TTT AGA TTG GAC CTG AGA ATA ACC ACC CCA AGG CAT AGG CCA CTA CTG GAT CTG - 3' | **Cox5a Down** (For construction of KanMX deletion cassette) |
| 13 | 5' - CGC CTC CCT ACG CTT C - 3' | **Cox5a Forward** (For checking correct chromosomal integration) |
| 14 | 5' - GCT CAG TAA GCT GTG CCC - 3' | **Cox5a Reverse** (For checking correct chromosomal integration) |
| 15 | 5' - TCT CCT TCA TTA CAG AAA CGG - 3' | **KanMX4 Forward** (For checking correct chromosomal integration) |
| 16 | 5' - CCG TTT CTG TAA TGA AGG AGA - 3' | **KanMX4 Reverse** (For checking correct chromosomal integration) |

The underlined sequences highlighted in Table 1 are those parts of the oligonucleotide which are complementary to the KanMX4 cassette.

## 1.1.4 Batch Fermentations

Organisms were grown in complete YPD medium [1% (w/v) yeast extract. 2% (w/v) peptone, 5% (w/v) glucose]. Respiratory deficiency was determined by growth on YEPG medium which contained 3% (w/v) glycerol as the sole carbon source.

Strain-specific ethanol productivity was determined in triplicate in batch cultures in 3L Bioengineering LKF2000 basic fermenters (Bioengineering AC, CH8636-Wald, Switzerland) with a working volume of 2L. Monitoring and control of fermentations were achieved using a "Pi" computer control system (Process Intelligence Ltd., Warwick, UK). Data from the variables described below was recorded at 2 min intervals throughout the course of the fermentations.

The cultures were agitated at 850 r.p.m. which was achieved using 2 x 6 blade Rushton impellers mounted on a central shaft and driven by a 250W VDE electric motor (Lenze, D-4923, Germany). Excessive foaming was prevented by the addition of several drops of a silicone-based anti-foaming agent (Mazu DF8005). Cultures were aerated at 1Lmin$^{-1}$ (1v/v/m) which was achieved using a rotameter and precision control valve. The fermenters were equipped with a 405-DPAS-50-K85/200 combination pH electrode and the pH of all fermentations was maintained at a value of 5.0 by the addition of 3M HCl and 3M NaOH solutions via W/M 501u pumps (Watson and Marlow, UK). Control of pH was implemented, via the "Pi" system, using an on/off control loop about the set point of pH 5.0 (±0.005). All fermentations were carried out at 30°C (±0.005°C) using the proportional (P) and Integral (I) controller of the "Pi" system. Dissolved oxygen in the medium was continuously monitored by means of a DO probe (Ingold, UK), as was $CO_2$ in the exhaust gas using an infra-red gas analyser (ADC Instruments Ltd., Hoddesden, UK). The various control systems of the fermenter were established and then inoculation followed with a 1% overnight dense seed culture. The fermentation was allowed to proceed for 45 hours and the $CO_2$ concentration in the exhaust gas was monitored constantly. Samples were taken from the middle of the exponential phase at 1 hour intervals right through to the end of the stationary phase and used for dry weight and Gas Chromatography analysis (GC).

Strain-specific ethanol tolerances were determined in batch cultures in specially adapted 100ml Erienmeyer flasks (called Klett Flasks) in combination with the Klett Summerson colorimeter and a green filter (Klett Summerson, USA). Cultures were grown to early exponential phase overnight and then split into 18ml aliquots to which 2ml of either sterile distilled water or ethanol solution were added to the aliquots giving final concentrations of 0, 2,3,4,6 and 10% (w/v) ethanol. Each concentration was carried out in triplicate and hourly measurements were made of cell density. A 1ml sample was extracted from the flask, diluted and plated onto YPD for a viable cell count. The remainder of the sample was centrifuged (15,000 rpm, 4°C, 5 min) and the supernatant frozen for subsequent analysis of ethanol concentration by GC.

### 1.1.5 Cell Dry Weight Determinations

A 20ml sample from the fermenter was centrifuged (15,000 rpm, 4"C, 5 min) and the supernatant decanted into a sterile container for the determination of ethanol concentrations. The cell pellet was washed once in 20ml sterile distilled water, the cells harvested again and dried at 90°C until a constant cell dry weight was achieved.

### 1.1.6 Measurement of Ethanol Concentration

Fermenter samples were stored at -20°C and assayed for ethanol content by gas chromatography using a PYE-UNICAM 204 series fitted with a carbowax column. The injector and FID were set at 200°C and the column maintained at 80°C. The flame gas was a mixture of air and hydrogen and the carrier gas was oxygen-free nitrogen (BOC Ltd.) running at 161b/in$^2$. A propan-1-ol internal standard was used for all samples and ethanol concentrations were calculated by the computer software. Ethanol concentrations were plotted against time and the specific rate of ethanol production ($q_p$) was determined at the steepest point [$q_p=r_p/x$].

## 1.2. RESULTS AND DISCUSSION

### 1.2.1. Ethanol Tolerance

All four members of the isogenic series (FY23WT, FY23Δ*pet191*, FY23Δ*cox5a* and FY23$\rho^0$) were grown in batch culture at 30°C and ethanol, at various concentrations, was introduced at the beginning of the exponential phase. Cell growth was followed for eight hours measuring optical densities with a Klett meter.

Upon addition of ethanol to an exponentially growing population of cells, there is an immediate drop in the growth rate. All four strains show this characteristic which is, at least partially, due to a dilution effect since it also occurs in the cultures dosed with only distilled water (i.e. 0% (w/v) ethanol). Graphs were plotted of loge Klett units against elapsed time and linear regression analysis was performed to calculate culture specific growth rates, μ. The effect of ethanol on the growth rate of the 4 members of the series was analysed by comparing relative growth rates at each ethanol concentration, and calculating these as a percentage of the growth rate in the absence of ethanol.

Ethanol inhibition kinetics are thought to follow those of non-competitive inhibition and can be modeled using a variant of the Monod equation. From the specific growth rate values calculated, it is possible to determine values of $K_i$ by inserting them into the rearranged equation [$\mu_m/\mu_i$ - 1 = I. 1/$K_i$]. It has been shown that simply using raw biomass accumulation data does not produce a straight line relationship. This is due to an irreversible effect (cell death) which renders the application of equilibrium kinetics invalid; cell viability must therefore be taken into account. The initial values of μ calculated are $\mu_{apparent}$ values determined from the growth of a culture containing both living and dead cells. For this reason, $\mu_{apparent}$ at each ethanol concentration. determined solely from Klett values, had to be corrected to $\mu_{true}$ using the viability data collected during each experiment.

TABLE 2-

| Table of Inhibition Constants | | |
|---|---|---|
| | $K_i$ (true growth rate) | |
| **Strain** | % (w/v) | M |
| **FY23WT** | 2.30 | 0.50 |
| **FY23Δ*pet191*** | 2.14 | 0.47 |
| **FY23Δ*cox5a*** | 1.94 | 0.42 |
| **FY23$\rho^0$** | 1.71 | 0.37 |

TABLE 3-

| Specific Growth Rates and Ethanol Production ($q_p$) | | |
|---|---|---|
| **STRAIN** | $\mu_{max}$ ($hr^{-1}$) (n) | $q_p$ (g ethanol/g dry biomass/hr) (n) |
| **FY23WT** | 0.422 ± 0.01 (3) | 0.939 ± 0.10 (3) |
| **FY23Δ*pet191*** | 0.403 ± 0.02 (3) | 1.339 ± 0.15 (3) |
| **FY23Δ*cox5a*** | 0.406 ± 0.01 (3) | 1.223 ± 0.05 (3) |
| **FY23$\rho^0$** | 0.271 ± 0.01 (3) | 0.893 ± 0.02 (2) |

TABLE 4-

| Specific Growth Rates and Ethanol Production ($q_p$) as percentage of FY23WT | | |
|---|---|---|
| **STRAIN** | $\mu_{max}$ (%) | $q_p$ (%) |
| **FY23WT** | 100.0 | 100.0 |
| **FY23Δ*pet191*** | 95.5 | 142.6 |
| **FY23Δ*cox5a*** | 96.2 | 130.2 |
| **FY23$\rho^0$** | 64.2 | 95.1 |

**1.2.2 The Effect of Ethanol on Cell Viability**

Pirt's (1975, "Principles of microbe and cell cultivation" Oxford University Press, Blackwell Scientific, Oxford, U.K.) equation, which takes into account the death of cells in a batch culture, was used to calculate the true specific growth rates at each ethanol concentration. This assumes that the rate of cell death in a batch culture is proportional to the number of viable cells present.

$$Y_T = y_{T(0)} + \frac{\mu . y_{v(0)}}{\mu - k}(e^{(\mu - k)t} - 1)$$

Corrected values of $\mu$ ($\mu_{true}$) were used in the adapted Monod equation and used to re-calculate $K_i$'s. Plotting [($\mu_{max(true)}/\mu_{i(true)}$) -1] against inhibitor concentration (i) yields a straight line with a gradient, $1/K_i$. In order to increase the accuracy of the plot, inhibitor concentrations plotted were the average of the ethanol concentration in each Klett flask over the course of the batch experiment. This was determined by GC analysis.

The higher the $K_i$, the more tolerant the strain is to ethanol inhibition of growth. Table 2 presents the $K_i$ values for the 4 strains and shows that the parent FY23WT is the most tolerant, and the mitochondrial petite is the most sensitive (76% the tolerance of FY23 WT). Analyses of the nuclear petites demonstrates that the 100% respiratory deficient nuclear petite, FY23Δ*pet191*, had an ethanol tolerance comparable to FY23WT ($K_i$ is approximately 95% of the wild-type value) and the 85% respiratory deficient FY23Δ*cox5a* had a tolerance 86% of that exhibited by the wild-type strain.

**1.2.3 Ethanol Production**

Batch fermentation was performed in a 2L working volume stirred vessel to compare the ethanol productivities of members of the isogenic series. In order to allow a good comparison of ethanol productivities, high fermentation rates were required and this was achieved by performing all fermentations under conditions of glucose repression. 5% (w/v) glucose. The medium was saturated with sterile air throughout the fermentation. This was maintained so that oxygen would be available to the yeast for non-respiratory functions during fermentation primarily the biosynthesis of sterols and unsaturated fatty acids, and also the biosynthesis of porphyrin and heme, the oxidation of medium components and the differentiation of promitochondria to mitochondria. Molecular oxygen would also be available for respiration by FY23WT and FY23Δ*cox5a* once the glucose substrate had been exhausted.

Figs 4 - 7 show fermentation data of the 4 members of the isogenic series. In each case, the concentration of carbon dioxide in the exit gas gives a profile which follows that of classical batch growth. Numerous terms have been use to describe the growth phase (15-25 hr in Figs 4 - 6, and 5-25 hr in Fig. 7) which is invariably characterised by high levels of fermentative activity and substantially repressed respiration. The term "respirofermentative" growth is favoured since it reflects the physiological state of the cells and emphasises the predominantly fermentative growth during which the culture is growing at its maximum specific growth rate, $\mu_{max}$. Tables 3 and 4 show the maximum specific growth rates of each of the strains and comparison with FY23WT shows that disruption of the ORFs *PET191* and *COX5a* have had no significant effect on this parameter. The specific growth rate of $\rho^0$, on the other hand, is 64.2% of that of FY23WT indicating that loss of the mitochondrial genome has had a considerable effect on growth of the cytoplasmic petite.

Dissolved oxygen tension (DOT) was set at 100% at the time of inoculation and also monitored. The decrease in DOT coinciding with the latter stages of growth represents oxygen requirements for both non-respiratory (all strains) and respiratory functions (FY23WT and FY23Δ*cox5a* only), after which it returns to 100%. For all strains, the growth rate of the culture decreases as the cells enter the diauxic lag phase as the concentration of the initial fermentable substrate, glucose, decreases finally to a minimum. In Fig. 4, growth occurs following exhaustion of the glucose substrate with a diauxic growth phase during which time FY23WT resorts to using the product of fermentation, ethanol, as its principal carbon and energy source. Cell growth is achieved using respiration whereby ethanol is oxidised to acetate via oxidative phosphorylation. FY23Δ*cox5a* shows evidence of a respiratory growth phase with ethanol being used as the secondary substrate but not to the same extent as for the grande FY23WT. Carbon dioxide levels in the diauxic phase do not reach values as high as those for the grande and DOT concentrations do not fall as low, indicating that levels of respiration are not as high for the mutant. This 10-15% residual respiratory activity is believed to be directly attributable to the chromosomal *COX5b* gene.

The $CO_2$ profiles for FY23Δ*pet191* and FY23$\rho^0$ for the end of batch growth show a concentration of 0% (±0.05%) and DOT levels are fixed at 100%, indicating that there is no diauxic respiratory growth phase. As expected, these 2 strains display the petite phenotype and are consequently unable to oxidise ethanol as an alternative energy source.

For all fermentations, hourly samples were extracted from the middle of the respirofermentative exponential phase through to the end of the "respiratory" growth phase, the cells being used for dry weight analysis and the supernatant being frozen at 20°C for GC analysis. Values for specific rates of ethanol production, $q_p$, were subsequently calculated from these data.

Tables 3 and 4 summarise the values of $q_p$ for each of the 4 members of the isogenic series. The lowest value of $q_p$ was obtained with the mitochondrial petite FY23$\rho^0$, confirming results of previous workers. The parental grande FY23WT displayed a slightly higher $q_p$ than FY23$\rho^0$ at the conditions tested, probably due to its increased $\mu_{max}$ and the presence of an intact mitochondrial genome maintaining its ethanol tolerance. The partially respiratory deficient nuclear petite FY23Δ*cox5a* displayed a productivity 30.2% higher, and the totally respiratory deficient petite FY23Δ*pet191* 42.6% higher, than the wild-type. Due to its inability to respire, FY23Δ*pet191* displayed an increased glycolytic flux from glucose through to ethanol. This was higher than the respiratory competent wild-type which preferred oxidative phosphorylation for the production of ATP. The relative productivity of FY23Δ*cox5a* was approximately 9% lower than that of FY23Δ*pet191* which roughly corresponds to the 10-15% residual respiratory activity provided by the *COX5b* gene.

### 1.2.4 Respiratory Quotients

The respiratory quotient (RQ), is defined as moles of $CO_2$ produced / moles of $O_2$ consumed.

For the complete oxidation of carbohydrate, i.e. full respiratory growth, the RQ is 6/6 = 1.0. However, in practice, yeast consumes oxygen for non-respiratory functions and so an RQ value of less than 1 is seen. In truly anaerobic, fermentative growth, no oxygen is consumed so the theoretical value of the RQ would be infinity.

In order to determine whether the four strains of the isogenic series are growing respiratively (low RQ values) or fermentatively (high RQ values), the RQ values over the course of each batch fermentation were calculated (see Table 5).

The average RQ values of the two 100% respiratory-deficient petites, FY23Δ *pet191* and FY23$\rho^0$, were comparable i.e. 17.0-17.5 ± 1.0. The RQ value for the respiratory-sufficient parent, FY23WT, was 8.0 ± 1.0, and that of the approximately 85% respiratory-deficient FY23Δ*cox5a* was 15 ± 1.0. As predicted by the theory, the respirofermentatively growing FY23WT has the lowest RQ value, the partially respirative, but predominantly fermentative growth, displayed by FY23Δ*cox5a* has a higher RQ value, and the two purely fermentative strains, FY23Δ*pet191* and FY23$\rho^0$, have the highest RQ values.

TABLE 5-

| Respiratory Quotients (RQ) of the Isogenic Series | |
|---|---|
| **STRAIN** | **RQ** |
| **FY23WT** | 8.0 ± 1.0 |
| **FY23Δ*pet191*** | 17.0-17.5 ± 1.0 |
| **FY23Δ*cox5a*** | 15 ± 1.0 |
| **FY23**$\rho^0$ | 17.0-17.5 ± 1.0 |

### 1.3. CONCLUSIONS

Examination of the effects of exogenous ethanol on the four strains demonstrated that functional mitochondria are essential to maintain the tolerance of yeast to ethanol, the cytoplasmic petite mutant exhibited the lowest tolerance of the series. The mitochondrial genome is thought to play a significant role in certain cell surface phenomena and hence determine, to an undefined extent, the ethanol tolerance of a strain.

The specific productivity data shows that the increased productivity exhibited by the two nuclear petites was a result of their inability to respire in the "respirofermentative" phase of batch growth, and of their relatively high tolerance to ethanol. Their respiratory deficient phenotype meant that the Pasteur effect was successfully circumvented allowing higher fermentation rates to be achieved compared to the respiratory sufficient wild-type. The possession of fully functional mitochondria served to maintain their ethanol tolerance and, hence, a high growth rate. Even though the cytoplasmic petite avoided the Pasteur effect, the lack of the mitochondrial genome prevented high fermentation rates from being achieved.

Therefore respiratory-deficient nuclear petites will be of use in the commercial production of ethanol (particularly in circumstances where the oxygen supply cannot be tightly controlled). Nuclear petites with 100% respiratory deficiency, are unable to grow in a diauxic growth phase and so will not metabolise the product of fermentation (ethanol) as their secondary substrate. This allows higher final titres of ethanol to be achieved.

In summary strain FY23Δ*pet191* exhibited:

(a) a maintained tolerance to ethanol courtesy of the fully functional mitochondrial genome;
(b) a 43% increase in specific ethanol productivity due to its insensitivity to the Pasteur effect (this indicates that a higher final ethanol ceiling may be achieved);
(c) a maximum specific growth rate ($\mu_{max}$) comparable to that of its respiratory-sufficient parent; and
(d) its respiratory-deficient phenotype rendered it unable to use ethanol or non-fermentable sugars as substrate upon exhaustion of the glucose substrate and therefore results in higher ethanol titres being achieved.

### EXAMPLE 2

The work carried out in Example 1 highlighted a number of advantageous characteristics displayed by the laboratory nuclear petite, FY23Δ*pet191*.

The beneficial features of a nuclear petite mutation were therefore introduced into a commercially applicable brewing strain, K1 (a respiratory-sufficient, highly ethanol tolerant, high alcohol producer), to produce a most preferred yeast strain according to the present invention.

## 2.1 METHODS

### 2.1.1 Yeast Strains Used

#### 2.1.1.1 K1 WT

K 1 WT, a diploid, grande [$\rho^+$],(Lallemand U.K. LTD, Fife, Scotland and produced by Danstar Ferment AG) was used in this Example as an industrial, respiratory competent parental yeast. K1 WT was used as a control yeast strain in fermentation studies and as the yeast strain from which respiratory deficient yeast cells were generated.

#### 2.1.1.3 K1Δ*pet*191ab

K1Δ*pet191ab* is a yeast cell according to the first aspect of the invention. K1Δ*pet191ab* is a nuclear petite mutant of K1 WT with disruption of both copies of the *PET191* gene This mutation causes failure of cytochrome c oxidase holoenzyme assembly and thereby induces 100% respiratory deficiency. Details concerning the production of the mutant are given below.

### 2.1.1 Genetic Modification of an Industrial Yeast Strain

K1 WT has 2 copies of *PET191* (i.e. it is diploid for that gene) and steps were therefore taken to delete both gene copies (*PET191*a and *PET191*b).

Multiple gene deletions in K1 WT were carried out using the modified Polymerase Chain Reaction (PCR)-mediated gene disruption technique developed by Güldener *et al.* (1996, Nucl. Acids. Res. (24) 13: p2519 - 2524) in which a *loxP-kanMX-loxP* disruption cassette combines the advantages of the heterologous *kan*$^R$ marker (Wach *et al*., 1994 *supra*), with those from the *Cre-loxP* recombination system of bacteriophage P1.

#### 2.1.1.1 Disruption of the first copy of *PET191(a)*

#### (a) Generation of a disruption cassette

The PCR method as described in 1.1.2.1 was initially used to produce a 1.36kb disruption cassette (*pet191*::**loxP**KanMX4(1)) which was used to make non-functional the ORF *PET191* in K1. The cassette was the same as that previously described except the KanMX4 gene was flanked by DNA encoding 34bp LoxP inverted repeats (SEQ ID NO. 17).

The disruption cassette was generated using the vector pUG6 (circular extrachromosomal DNA element described by Guldener *et al*., *supra*) as template and the same two primers used in 1.1.2.1 (SEQ ID NOs 7 and 8).

pUG6 comprises the KanMX4 gene (from *E.coli* which may be expressed in yeast to render said yeast resistant to the antibiotic geneticin) flanked by 34bp loxP inverted repeats (see Fig. 8).

The two primers had 19-22 nucleotide homology to regions either side of the loxPKanMX4 cassette and 35 nucleotide extensions which were either homologous to the region immediately downstream of the start codon or to that upstream of the stop codon of the ORFs *PET191* (see Fig 9(a)).

#### (b) Transformation of K1 with the Disruption cassette

Following correct amplification of the cassette, 1-2μg of ethanol precipitated DNA was used in the transformation of K1WT. Upon transformation, recombination events took place between the homologous regions of the PET191::**LoxP**KanMX4(1) cassette and the target gene resulting in the disruption of the *PET191* (Fig 9 (b)). Due to the geneticin resistant gene of the disruption cassette, transformant selection took place on geneticin plates and transformant screening for correct gene disruptants followed.

#### (c) Verification of Correct Gene Disruption

Correct targeting of the disruption cassette into the geneticin resistant yeast genomic locus was verified by analytical PCR (Wach *et al*., 1994) as described in 1.1.3.

The results of analytical PCR indicated that the industrial strain, K1Δ*pet191a*, (the "a" designates the disruption of the first copy of *PET191* in K1) had one of the two *PET191* alleles correctly disrupted but the other *PET191* ORF (*PET191*b) remained intact i.e. the strain was heterozygous at the *PET191* locus on chromosome X.

K1Δ*pet191a* was also subjected to pulsed-field gel electrophoresis using the Contour-clamped Homogenous Electric Field (CHEF) technique in order to separate the chromosomes. This was followed by Southern hybridisation whereby a $^{32}$P-labelled loxPKanMX4 cassette was used to probe a nitrocellulose membrane (with chromosomal DNA bound to it). Subsequent exposure to a phosphoimager (Biorad, UK) indicated that the pet191::loxPkanMX4 cassette had integrated correctly into chromosome X.

### (d) Construction of YEP351-cre-cyh

YEP351cre-cyh is derived from YEP351 (fully described by Hill *et al*., Yeast 2:163-167 1986) as illustrated in Fig 10. YEP351cre-cyh is a multi-copy plasmid comprising the *GAL1*/cre recombinase system, and a marker gene conferring resistance to the antibiotic cycloheximide (cyh). Cycloheximide is an antibiotic which inhibits protein synthesis and, in eukaryotes, this is brought about by the inhibition of peptidyl transferase activity of the 60S ribosomal subunit of the 80S ribosome. *S. cerevisiae* is sensitive to low concentrations of cycloheximide, typically 0.2μg/ml.

A 3300bp *Xba*I-*Hind*III fragment of the cyclohexamide gene (Genbank Accession No. M64932 and Sasnauskas *et al.* (1992), Gene 116 p105-108) was ligated into the *Xba*I-*Hind*III digested yeast multicopy plasmid YEP351 (5644bp) giving YEP351-cyh (8944bb) (see Fig 10(b)). *E.coli* (XL1-Blue) was transformed with the resulting ligation mix, and disruption of the lacZ gene of YEP351 made the isolation of putative YEP351-cyh clones possible using the blue/white selection system. Miniprep isolation of the plasmid from *E.coli*, and its introduction into *S.cerevisiae*, FY23, rendered the strain resistant to the antibiotic cycloheximide (2μg/ml).

The 2260bp *Pvu*II "cre recombinase" fragment consisting of the *GAL1* promoter, cre recombinase gene and *CYC1* terminator from pSH47 (see Güldener *et al., supra*) was ligated into *Sac*I digested, blunt-ended YEP351-cyh giving YEP351-cre-cyh (11204bp) (see Fig. 10(c)). *S.cerevisiae* was transformed with the resulting ligation mix, and putative YEP351-cre-cyh clones were subjected to, and isolated by. yeast colony hybridisation using 32P-labelled "cre" insert as a probe.

### (e) Marker Retrieval

In order to delete the second *PET191* allele (*PET191b*) from the geneticin resistant heterozygous deletant strain K1Δ*pet191a*, it was first necessary to remove the KanMX marker from *PET191a* using the cre recombinase system. This was achieved by initially transforming the strain with YEP351cre-cyh (see Fig 9 (d) and Fig. 10). Selective pressure of the vector was maintained on K1Δ*pet191a* by supplementing minimal media with cycloheximide (cyh = 2μg/ml).

Expression of the cre recombinase gene was achieved in the presence of galactose acting as the sole carbon source (see Fig 9 (e)). The cre recombinase enzyme mediates the recombination of the two inverted repeat *loxP* sites which flank the *KanMX* marker. The 2 loxP sites line up next to one another and recombine resulting in the efficient excision of the marker from the disrupted *PET191* locus, allowing for its repeated use in subsequent gene deletions. This technique ensures the removal of virtually all heterologous DNA used in gene deletions which is important due to the current constraints of using genetically engineered yeasts in the brewing industry. One of the two 34bp loxP sites remains at the deleted *PET191* locus (see Fig 9 (f)). Growth for only 2 hr in galactose medium was sufficient for loxPKanMX4 marker excision in >95% of the cells, with the resulting colonies becoming geneticin sensitive. This was confirmed by plating cells onto minimal media supplemented with cycloheximide, and transferring colonies onto geneticin and witnessing no growth.

Correct marker excision from K1Δ*pet191a* was verified by colony PCR which resulted in the production of the 572bp wild-type band only. The amplification of a deletion fragment was no longer possible since the previously integrated KanMX cassette was now absent rendering the strain geneticin-sensitive.

### 2.1.1.2 Disruption of the Second copy of *PET191*

A second round of gene disruption was then conducted in the geneticin-sensitive strain containing YEP351-cre-cyh by transforming with a second pet191::loxPKanMX4(2) disruption cassette (Fig. 11(2)).

pet191::loxPKanMX4(2) was constructed as described in 2.1.1.1 except different primers were used to construct this cassette such that the 5' and 3' ends of the disruption cassette were homologous with regions of the *PET191* gene which were internal to the regions of homology of the first disruption cassette. The two primers used in the PCR step (SEQ ID NO 18 and 19) had nucleotide homology to regions either side of the loxPKanMX4 cassette and nucleotide extensions which were homologous to the internal regions of the *PET191* gene.

**TABLE 6 Primers used for the generation of the second disruption cassette, Pet191::loxPKanMX4 (2).**

| Oligonucleotide Primer DNA SEQ. ID No. | DNA sequence | Description |
|---|---|---|
| 18 | 5'- GAG ATC GCC CTG TGT CAT GAT AGA GAG ACA TAA CCC TCA AGA CAG C -3' | **PET191 Up (2)** (For construction of KanMX deletion cassette) |
| 19 | 5'- TTT ATT TAT GTA TAT ATT TAC AGG CCA ATT TTC ATA AAT ACA TAG GCC -3' | **PET191 Down (2)** (For construction of KanMX deletion cassette) |

Colonies resistant to 0.5mg/ml G418 were subjected to colony PCR. The sole appearance of the 824bp deletion fragment verified the possession of a double *PET191* deletant, K1Δ*pet191ab* (the "b" designates the disruption of the second copy of an unknown number of *PET191* ORFs in K1). No 572bp wild-type fragment was seen confirming that both copies of *PET191* had been successfully deleted (Fig. 9(f))

As for 2.1.1.1(f), all heterologous DNA (except for a single 34bp loxP site) was excised from the mutant, to give K1Δ*pet191ab* (as defined above) by expression of the cre recombinase on galactose medium resulting in the strain's sensitivity to geneticin.

**2.1.1.3 Vector Retrieval**

The vector YEP351-cre-cyh which rendered the strain resistant to cycloheximide was removed by growing the strain for at least 100 generations with a vector loss rate of 1% per generation. Replica plating of the culture after about 10 days of repeated subculturing (100μl of an overnight culture into 100ml YPD) resulted in a geneticin-sensitive, cycloheximide-sensitive K1Δ*pet191ab* nuclear petite.

**2.1.2 Summary of K1Δ*pet191ab***

The industrial yeast strain, K1WT, was genetically engineered to produce the respiratory-deficient, nuclear petite, K1Δ*pet191ab*. K1Δ*pet191ab* is isogenic (genetically identical) to its parent, K1WT, except for the deletion of the two genes of *PET191*, which are required for respiration. All that remains inserted at each deleted *PET191* locus is a single 34bp loxP site. Analytical PCR is a highly specific method used to check for correct gene deletion and verified:

(a) the absence of both copies of *PET191*, and
(b) that no other non-target genes had been altered.

The nuclear petite possesses fully functional mitochondrial DNA and is not resistant to any antibiotics such as geneticin or cycloheximide as the KanMX gene and the vector YEP351-cre-cyh were removed. Lack of growth on both of these antibiotics verified the yeast's sensitivity.

It will be appreciated that mutants of K1 in which the marker gene is not deleted will also fall within the scope of the invention. For instance, the DNA fragment of SEQ ID NO. 3 (the LoxP::KanMX4 DNA fragment inserted during homologous recombination) may be left in one or both copies of *PET191* in K1. When this is the case the nuclear petite yeast is still suitable for producing ethanol according to the method of the invention despite the fact that excision of the marker gene is often desirable (e.g. to prevent the introduction of micro-organisms with antibiotic resistance into the environment).

**2.2 Characterization of K1Δ*pet191ab***

K1Δ*pet191ab* cells may be grown essentially according to the methods described in Example land ethanol produced from said cells may be assessed.

## Claims

**1.** A method of producing ethanol comprising growing respiratory deficient yeast cells which have at least one nuclear gene, or product thereof, required for respiration which is non-functional and/or inhibited in a growth medium and separating ethanol from said yeast cells.

**2.** The method according to claim 1 wherein the respiratory deficient yeast cell is derived from a respiratory sufficient yeast strain.

**3.** The method according to claim 2 wherein respiration in the respiratory deficient yeast cell is reduced by at least 60%. relative to a respiratory sufficient yeast strain.

**4.** The method according to claim 2 wherein respiration in the respiratory deficient yeast cell is reduced by at least 75%, relative to a respiratory sufficient yeast strain.

**5.** The method according to claim 2 wherein respiration in the respiratory deficient yeast cell is reduced by at least 85%, relative to a respiratory sufficient yeast strain.

**6.** The method according to claim 2 wherein respiration in the respiratory deficient yeast cell is reduced by at least 95%, relative to a respiratory sufficient yeast strain.

**7.** The method according to any one of claims 2 to 6 wherein the respiratory deficient yeast cell is generated by genetic modification of a respiratory sufficient yeast strain such that the at least one nuclear gene, or product thereof, required for respiration is non-functional.

**8.** The method according to claim 7, wherein the modification is a mutation which disrupts the expression or function of the gene product.

**9.** The method according to claim 8, wherein the mutation is introduced by homologous recombination between the nuclear gene and a DNA molecule.

**10.** The method according to claims 9, wherein the respiratory deficient yeast cell is generated using a PCR-mediated gene disruption technique.

**11.** The method according to any preceding claim, wherein the nuclear gene is a *PET* gene.

**12.** The method according to claim 11, wherein the gene is *PET191.*

**13.** The method according to claim 12, wherein the *PET191* gene is modified such that the DNA sequence identified as SEQ ID NO. 1 is excised from the yeast cell's genome and replaced with the DNA sequence identified as SEQ ID NO. 2.

**14.** The method according to claim 12. wherein the *PET191* gene is modified such that the DNA sequence identified as SEQ ID NO. 1 is excised from the yeast cell's genome and replaced with the DNA sequence identified as SEQ ID NO. 17.

**15.** The method according to claim 12, wherein the *PET191* gene is modified such that the DNA sequence identified as SEQ ID NO. 4 is excised from the yeast cell's genome and replaced with the DNA sequence identified as SEQ ID NO. 17.

**16.** The method according to any one of claims 1 to 10, wherein the nuclear gene which is modified is associated with the function or expression of cytochrome c oxidase.

**17.** The method according to claim 14, wherein the nuclear gene which is modified is *COX5a.*

**18.** The method according to claim 17, wherein the *COX5a* gene is modified such that the DNA sequence identified as SEQ ID NO. 5 is excised from the yeast cell's genome and replaced with the DNA sequence identified as SEQ ID NO. 2.

**19.** The method according to any one of claims 1 to 6, wherein at least one nuclear gene product required for respiration is non-functional and/or inhibited.

**20.** The method according to any preceding claim, wherein the respiratory deficient yeast cell is *Saccharomyces cerevisiae* or a derivative thereof.

**21.** The method according to any one of claims 1 to 19, wherein the respiratory deficient yeast cell is *S. uvarum (S. carlsbergensis)* or a derivative thereof.

**22.** The method according to any one of claims 1 to 19, wherein the respiratory deficient yeast cell is a member of the *Saccharomyces sensu stricto* group or a derivative thereof.

**23.** The method according to any one of claims 1 to 19, wherein the respiratory deficient yeast cell is FY23 or a derivative thereof.

**24.** The method according to any one of claims 1 to 19, wherein the respiratory deficient yeast cell is K1 or a derivative thereof.

**25.** The method according to any one of claims 1 to 19, wherein the respiratory deficient yeast cell is selected from the group consisting of an M-strain or a derivative thereof, Red Star or a derivative thereof, Oenological Yeast U.C.L.M. S325 or a derivative thereof or Oenological Yeast U.C.L.M. S377 or a derivative thereof.

**26.** The method according to any preceding claim, wherein the growth medium contains a fermentable sugar as a source of carbohydrate.

**27.** The method according to any preceding claim, wherein the growth medium is YPD.

**28.** The method according to any preceding claim, wherein the cells are grown in a fermenter with means for the regulation of at least one of temperature, air supply, nutrient content, waste removal and product extraction.

**29.** The method according to any preceding claim, wherein the respiratory deficient yeast are grown by batch culture.

**30.** The method according to any one of claims 1 to 28. wherein the respiratory deficient yeast are grown by continuous culture.

**31.** The method according to any preceding claim for the production of fuel ethanol.

**32.** A yeast cell **characterised in that** the *PET191* gene is functionally deleted.

**33.** The yeast cell according to claim 32 wherein the *PET191* gene is functionally deleted by homologous recombination between a DNA molecule and the DNA encoding the *PET191* gene.

**34.** The yeast cell according to claim 32 or 33 wherein the yeast is a strain of FY23.

**35.** The yeast cell according to claim 34 wherein the *PET191* gene is functionally deleted by excision of the DNA sequence identified as SEQ ID NO. I from the yeast cell's genome and replaced with the DNA sequence identified as SEQ ID NO. 2.

**36.** The yeast cell according to claim 32 or 33 wherein the yeast is a strain of K1.

**37.** The yeast cell according to claim 36 wherein one copy of the *PET191* gene (*PET191a*) has the DNA sequence identified as SEQ ID NO. 1 excised and replaced with the DNA sequence identified as SEQ ID NO. 17 and the second copy of *PET191* (*PET191b*) has the DNA sequence identified as SEQ ID NO. 4 excised and replaced with the DNA sequence identified as SEQ ID NO. 17.

**Patentansprüche**

**1.** Verfahren zur Herstellung von Ethanol, welches das Wachsen von Hefezellen mit Atmungsdefekt, welche wenigstens ein für die Atmung erforderliches Kerngen oder ein Produkt davon haben, welches nicht funktionell und/oder inhibiert ist, in einem Wachstumsmedium, und die Abtrennung von Ethanol von den Hefezellen umfasst.

**2.** Verfahren nach Anspruch 1, wobei die Hefezelle mit Atmungsdefekt von einem atmungsfähigen Hefestamm abstammt.

**3.** Verfahren nach Anspruch 2, wobei die Atmung in der Hefezelle mit Atmungsdefekt im Vergleich zu einem atmungsfähigen Hefestamm um wenigstens 60% reduziert ist.

**4.** Verfahren nach Anspruch 2, wobei die Atmung in der Hefezelle mit Atmungsdefekt im Vergleich zu einem atmungsfähigen Hefestamm um wenigstens 75% reduziert ist.

**5.** Verfahren nach Anspruch 2, wobei die Atmung in der Hefezelle mit Atmungsdefekt im Vergleich zu einem atmungsfähigen Hefestamm um wenigstens 85% reduziert ist.

6. Verfahren nach Anspruch 2, wobei die Atmung in der Hefezelle mit Atmungsdefekt im Vergleich zu einem atmungsfähigen Hefestamm um wenigstens 95% reduziert ist.

7. Verfahren nach einem der Ansprüche 2 bis 6, wobei die Hefezelle mit Atmungsdefekt durch derartige genetische Modifikation eines atmungsfähigen Hefestammes erzeugt wird, dass wenigstens ein für die Atmung erforderliches Kerngen oder ein Produkt davon nicht funktionell ist.

8. Verfahren nach Anspruch 7, wobei die Modifikation eine Mutation ist, welche die Expression oder Funktion des Genprodukts stört.

9. Verfahren nach Anspruch 8, wobei die Mutation durch homologe Rekombination zwischen dem Kerngen und einem DNA-Molekül eingeführt wird.

10. Verfahren nach Anspruch 9, wobei die Hefezelle mit Atmungsdefekt unter Verwendung einer PCR-vermittelten Genstörungstechnik erzeugt wird.

11. Verfahren nach einem der vorhergehenden Ansprüche, wobei das Kerngen ein *PET*-Gen ist.

12. Verfahren nach Anspruch 11, wobei das Gen *PET191* ist.

13. Verfahren nach Anspruch 12, wobei das *PET191*-Gen derartig modifiziert wird, dass die als SEQ ID NO. 1 bezeichnete DNA-Sequenz aus dem Genom der Hefezelle ausgeschnitten und durch die als SEQ ID NO. 2 bezeichnete DNA-Sequenz ersetzt wird.

14. Verfahren nach Anspruch 12, wobei das *PET191-Gen* derartig modifiziert wird, dass die als SEQ ID NO. 1 bezeichnete DNA-Sequenz aus dem Genom der Hefezelle ausgeschnitten und durch die als SEQ ID NO. 17 bezeichnete DNA-Sequenz ersetzt wird.

15. Verfahren nach Anspruch 12, wobei das *PET191*-Gen derartig modifiziert wird, dass die als SEQ ID NO. 4 bezeichnete DNA-Sequenz aus dem Genom der Hefezelle ausgeschnitten und durch die als SEQ ID NO. 17 bezeichnete DNA-Sequenz ersetzt wird.

16. Verfahren nach einem der Ansprüche 1 bis 10, wobei das modifizierte Kerngen mit der Funktion oder Expression der Cytochrom c Oxidase verbunden ist.

17. Verfahren nach Anspruch 14, wobei das modifizierte Kerngen *COX5a* ist.

18. Verfahren nach Anspruch 17, wobei das *COX5a*-Gen derartig modifiziert wird, dass die als SEQ ID NO. 5 bezeichnete DNA-Sequenz aus dem Genom der Hefezelle ausgeschnitten und durch die als SEQ ID NO. 2 bezeichnete DNA-Sequenz ersetzt wird.

19. Verfahren nach einem der Ansprüche 1 bis 6, wobei wenigstens ein für die Atmung erforderliches Genprodukt nicht funktionell und/oder inhibiert wird.

20. Verfahren nach einem der vorhergehenden Ansprüche, wobei die Hefezelle mit Atmungsdefekt *Saccharomyces cerevisiae* oder ein Abkömmling davon ist.

21. Verfahren nach einem der Ansprüche 1 bis 19, wobei die Hefezelle mit Atmungsdefekt *S. uvarum (S. carlsbergensis)* oder ein Abkömmling davon ist.

22. Verfahren nach einem der Ansprüche 1 bis 19, wobei die Hefezelle mit Atmungsdefekt ein Mitglied der *Saccharomyces sensu* stricto-Gruppe oder ein Abkömmling davon ist.

23. Verfahren nach einem der Ansprüche 1 bis 19, wobei die Hefezelle mit Atmungsdefekt FY23 oder ein Abkömmling davon ist.

24. Verfahren nach einem der Ansprüche 1 bis 19, wobei die Hefezelle mit Atmungsdefekt K1 oder ein Abkömmling davon ist.

**25.** Verfahren nach einem der Ansprüche 1 bis 19, wobei die Hefezelle mit Atmungsdefekt aus der Gruppe bestehend aus einem M-Stamm, oder einem Abkömmling davon, Red Star, oder einem Abkömmling davon, der Weinhefe U. C.L.M. S325, oder einem Abkömmling davon, oder der Weinhefe U.C.L.M. S377, oder einem Abkömmling davon, ausgewählt wird.

**26.** Verfahren nach einem der vorhergehenden Ansprüche, wobei das Wachstumsmedium einen fermentierbaren Zucker als eine Kohlenhydratquelle enthält.

**27.** Verfahren nach einem der vorhergehenden Ansprüche, wobei das Wachstumsmedium YPD ist.

**28.** Verfahren nach einem der vorhergehenden Ansprüche, wobei die Zellen in einem Fermenter mit einer Regeleinrichtungen für zumindest Temperatur oder Luftzufuhr oder Nahrungsgehalt oder Abfallentfernung oder Produktextraktion wachsen.

**29.** Verfahren nach einem der vorhergehenden Ansprüche, wobei die Hefen in einer diskontinuierlichen Kultur wachsen.

**30.** Verfahren nach einem der Ansprüche 1 bis 28, wobei die Hefen mit Atmungsdefekt in einer kontinuierlichen Kultur wachsen.

**31.** Verfahren nach einem der vorhergehenden Ansprüche für die Herstellung von Kraftsprit.

**32.** Hefezelle, **dadurch gekennzeichnet, dass** das *PET191*-Gen funktionell deletiert ist.

**33.** Hefezelle nach Anspruch 32, wobei das *PET191*-Gen durch homologe Rekombination zwischen einem DNA-Molekül und der für das *PET191*-Gen kodierenden DNA funktionell deletiert ist.

**34.** Hefezelle nach Anspruch 32 oder 33, wobei die Hefe ein Stamm von FY23 ist.

**35.** Hefezelle nach Anspruch 34, wobei das *PET191*-Gen durch Ausschnitt der als SEQ ID NO. 1 bezeichneten DNA-Sequenz aus dem Genom der Hefezelle funktionell deletiert und durch die als SEQ ID NO. 2 bezeichnete DNA-Sequenz ersetzt ist.

**36.** Hefezelle nach Anspruch 32 oder 33, wobei die Hefe ein Stamm von K1 ist.

**37.** Hefezelle nach Anspruch 36, wobei eine Kopie des *PET191*-Gens (*PET191a*), die eine als SEQ ID NO. 1 bezeichnete DNA-Sequenz hat, ausgeschnitten und durch die als SEQ ID NO. 17 bezeichnete DNA-Sequenz ersetzt ist, und die zweite Kopie von *PET191 (PET191b)*, die eine als SEQ ID NO. 4 bezeichnete DNA-Sequenz hat, ausgeschnitten und durch die als SEQ ID NO. 17 bezeichnete DNA-Sequenz ersetzt ist.

## Revendications

**1.** Procédé de production d'éthanol comprenant la mise en culture de cellules de levure qui présentent une déficience respiratoire et dont au moins un gène nucléaire, ou produit d'un tel gène, nécessaire à la respiration est non fonctionnel et/ou inhibé dans un milieu de croissance, et la séparation de l'éthanol desdites cellules de levure.

**2.** Procédé selon la revendication 1, dans lequel la cellule de levure présentant une déficience respiratoire est dérivée d'une souche de levure qui est suffisante pour ce qui est de la respiration.

**3.** Procédé selon la revendication 2, dans lequel la respiration dans la cellule de levure présentant une déficience respiratoire est réduite d'au moins 60 % par rapport à une souche de levure à respiration suffisante.

**4.** Procédé selon la revendication 2, dans lequel la respiration dans la cellule de levure présentant une déficience respiratoire est réduite d'au moins 75 % par rapport à une souche de levure à respiration suffisante.

**5.** Procédé selon la revendication 2, dans lequel la respiration dans la cellule de levure présentant une déficience respiratoire est réduite d'au moins 85 % par rapport à une souche de levure à respiration suffisante.

6. Procédé selon la revendication 2, dans lequel la respiration dans la cellule de levure présentant une déficience respiratoire est réduite d'au moins 95 % par rapport à une souche de levure à respiration suffisante.

7. Procédé selon l'une quelconque des revendications 2 à 6, dans lequel la cellule de levure présentant une déficience respiratoire est produite par modification génétique d'une souche de levure à respiration suffisante, de telle sorte que ledit au moins un gène nucléaire, ou produit d'un tel gène, nécessaire à la respiration est non fonctionnel.

8. Procédé selon la revendication 7, dans lequel la modification est une mutation qui perturbe l'expression ou le fonctionnement du produit génique.

9. Procédé selon la revendication 8, dans lequel la mutation est introduite par recombinaison homologue entre le gène nucléaire et une molécule d'ADN.

10. Procédé selon la revendication 9, dans lequel la cellule de levure présentant une déficience respiratoire est produite en utilisant une technique de disruption génique basée sur la PCR.

11. Procédé selon l'une quelconque des précédentes revendications, dans lequel le gène nucléaire est un gène *PET*.

12. Procédé selon la revendication 11, dans lequel le gène est le *PET191*.

13. Procédé selon la revendication 12, dans lequel le gène *PET191* est modifié de telle sorte que la séquence d'ADN identifiée comme SEQ ID NO. 1 est excisée du génome de la cellule de levure et remplacée par la séquence d'ADN identifiée comme SEQ ID NO. 2.

14. Procédé selon la revendication 12, dans lequel le gène *PET191* est modifié de telle sorte que la séquence d'ADN identifiée comme SEQ ID NO. 1 est excisée du génome de la cellule de levure et remplacée par la séquence d'ADN identifiée comme SEQ ID NO. 17.

15. Procédé selon la revendication 12, dans lequel le gène *PET191* est modifié de telle sorte que la séquence d'ADN identifiée comme SEQ ID NO. 4 est excisée du génome de la cellule de levure et remplacée par la séquence d'ADN identifiée comme SEQ ID NO. 17.

16. Procédé selon l'une quelconque des revendications 1 à 10, dans lequel le gène nucléaire qui est modifié est associé au fonctionnement ou à l'expression de la cytochrome c oxydase.

17. Procédé selon la revendication 14, dans lequel le gène nucléaire qui est modifié est le *COX5a*.

18. Procédé selon la revendication 17, dans lequel le gène *COX5a* est modifié de telle sorte que la séquence d'ADN identifiée comme SEQ ID NO. 5 est excisée du génome de la cellule de levure et remplacée par la séquence d'ADN identifiée comme SEQ ID NO. 2.

19. Procédé selon l'une quelconque des revendications 1 à 6, dans lequel au moins un produit de gène nucléaire nécessaire à la respiration est non fonctionnel et/ou inhibé.

20. Procédé selon l'une quelconque des précédentes revendications, dans lequel la cellule de levure présentant un déficit respiratoire est *Saccharomyces cerevisiae* ou un dérivé de *Saccharomyces cerevisiae*.

21. Procédé selon l'une quelconque des revendications 1 à 19, dans lequel la cellule de levure présentant une déficience respiratoire est *S. uvarum (S. carlsbergensis*) ou un dérivé de *S. uvarum* (*S. carlsbergensis).*

22. Procédé selon l'une quelconque des revendications 1 à 19, dans lequel la cellule de levure présentant une déficience respiratoire est un membre du groupe *Saccharomyces sensu stricto* ou un dérivé d'un membre du groupe *Saccharomyces sensu stricto.*

23. Procédé selon l'une quelconque des revendications 1 à 19, dans lequel la cellule de levure présentant une déficience respiratoire est FY23 ou un dérivé de FY23.

24. Procédé selon l'une quelconque des revendications 1 à 19, dans lequel la cellule de levure présentant une déficience respiratoire est K1 ou un dérivé de K1.

**25.** Procédé selon l'une quelconque des revendications 1 à 19, dans lequel la cellule de levure présentant une déficience respiratoire est sélectionnée dans le groupe consistant en une souche M ou un dérivé de souche M, Red Star ou un dérivé de Red Star, la levure oenologique U.C.L.M. S325 ou un dérivé de la levure oenologique U.C. L.M. S325 ou la levure oenologique U.C.L.M. S377 ou un dérivé de la levure oenologique U.C.L.M. S377.

**26.** Procédé selon l'une quelconque des précédentes revendications, dans lequel le milieu de croissance contient un sucre fermentable comme source d'hydrates de carbone.

**27.** Procédé selon l'une quelconque des précédentes revendications, dans lequel le milieu de croissance est le YPD.

**28.** Procédé selon l'une quelconque des précédentes revendications, dans lequel les cellules sont cultivées dans un fermenteur doté de moyens de régulation d'au moins un facteur parmi la température, l'alimentation en air, la teneur en nutriments, l'élimination des déchets et l'extraction du produit.

**29.** Procédé selon l'une quelconque des précédentes revendications, dans lequel la levure présentant une déficience respiratoire est cultivée en discontinu.

**30.** Procédé selon l'une quelconque des revendications 1 à 28, dans lequel la levure présentant une déficience respiratoire est cultivée en continu.

**31.** Procédé selon l'une quelconque des précédentes revendications pour la production d'éthanol pour carburant.

**32.** Cellule de levure **caractérisée par le fait que** le gène *PET191* est fonctionnellement délété.

**33.** Cellule de levure selon la revendication 32, dans laquelle le gène *PET191* est fonctionnellement délété par recombinaison homologue entre une molécule d'ADN et l'ADN codant pour le gène *PET191*.

**34.** Cellule de levure selon la revendication 32 ou 33, dans laquelle la levure est une souche de FY23.

**35.** Cellule de levure selon la revendication 34, dans laquelle le gène *PET191* est fonctionnellement délété par excision de la séquence d'ADN identifiée comme SEQ ID NO. 1 du génome de la cellule de levure et remplacé par la séquence d'ADN identifiée comme SEQ ID NO. 2.

**36.** Cellule de levure selon la revendication 32 ou 33, dans laquelle la levure est une souche de K1.

**37.** Cellule de levure selon la revendication 36, dans laquelle une copie du gène *PET191* (*PET191a*) présente une séquence d'ADN identifiée comme SEQ ID NO. 1 excisée et remplacée par la séquence d'ADN identifiée comme SEQ ID NO. 17 et la seconde copie de *PET191* (*PET191b*) présente la séquence d'ADN identifiée comme SEQ ID NO.4 excisée et remplacée par la séquence d'ADN identifiée comme SEQ ID NO. 17.

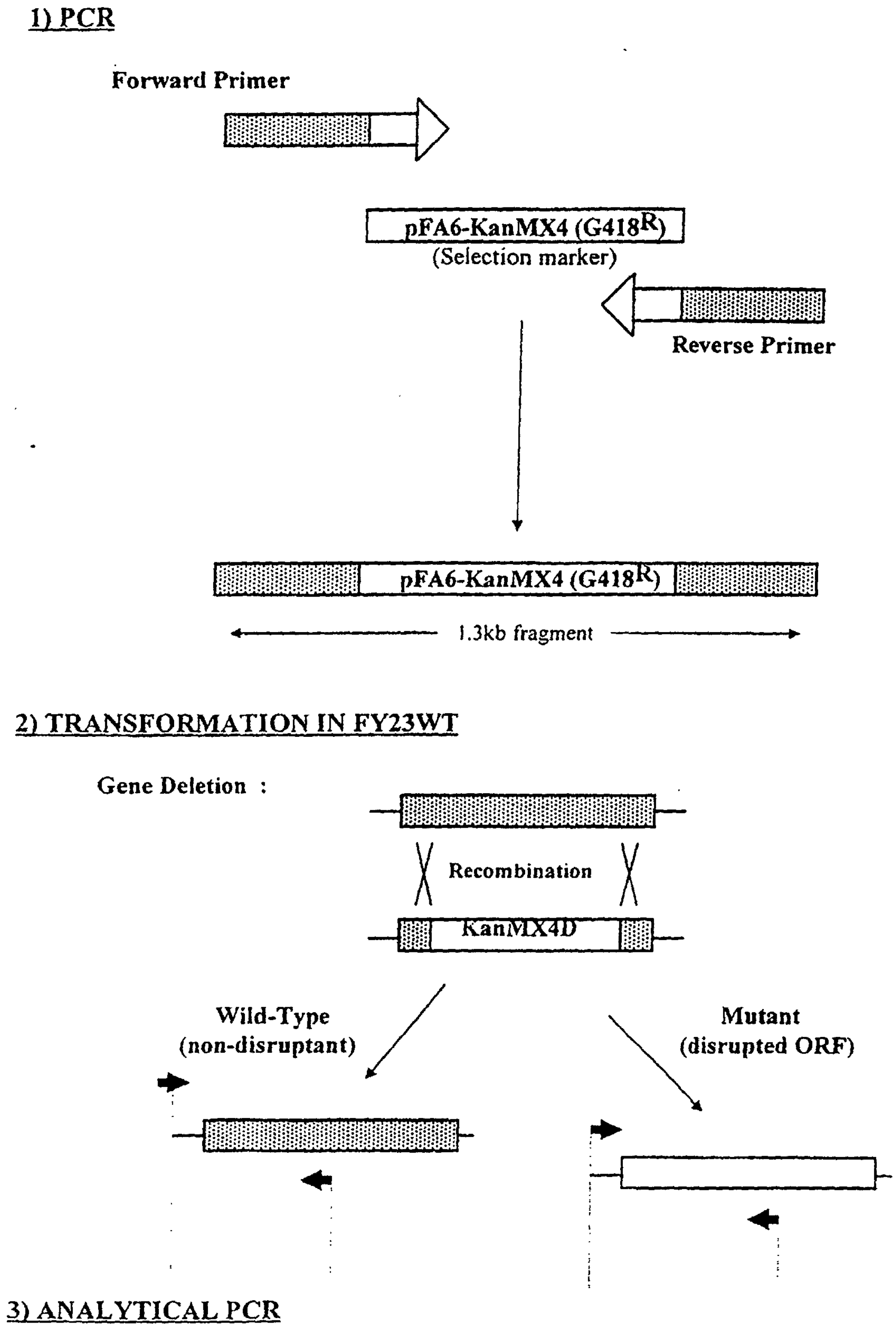
1) PCR
Forward Primer
pFA6-KanMX4 (G418R)
(Selection marker)
Reverse Primer
pFA6-KanMX4 (G418R)
1.3kb fragment
2) TRANSFORMATION IN FY23WT
Gene Deletion :
Recombination
KanMX4D
Wild-Type
(non-disruptant)
Mutant
(disrupted ORF)
3) ANALYTICAL PCR

FIG. 1

5'- gatatcaaaga**atg**gtagctagttgtaaagatcagaagaaggctgtcgct (PET 191 Up)

atatgtt↓tgcagagatcgccctgtgtcatgatagagagacataaccctca

agaatgtcttgacaatccagagctgaataaggatctgcccgaactttgta

ttgctcagatgaaagcatttttggattgtaagcgaggaatcgtcgacatg

actaagcggttcacaggtaacgcacctctgtcgactggcaagtacgatca (PET 191 Forward)

acagtacgaaaacttgtgcaaaggaaagtttgatccgagggaggagatgg

aaaagctaaaacttctgaacagccagcagaagg**act**gattatttatgaaa (Stop Codon)

attggcctgtaaatatatacataaataaatatat↓gcgtcaagctctttta (PET 191 Down)

ttcggtttgtccagtaatcctcttacaatatatatatatatatacgca

tgtagcgtattcaagaacattacatttaaaaaatagagggtagaacaata

aaaatgcacttaacaagttccgtaagtagaaattataatcagtctaagta

caaatttataccttagcttcatttcagttaagggcgatgcaataaaagtg

gaagaaaaaaaaagaagggaagaggtttgataatatatatatatatat

atataacattgctcagagaaacatacatcagtgatggaagataaagagca

gcaagacaatgcgaaacttgaaaacaatgagtcactaaaagatctgggag (PET 191 Reverse)

ttaacgtgctttcccaaagcagtttggaggaaaaaatagctaatgatgta

actaactttagcaacctacaatcgctgcagcaagaagagactcgtctaga - 3'

FIG.2

5'- aatctttatgactatcagatctgcaggcagaccgcctccctacgcttcta ( Cox5a Forward)

aatagaatgtacttagcacttctgcctacatagtaattattcatcattgt

aagtgtcacattgcaaaaatcgtccaatagacgttttcctcgtttgtgat

tggccctacgttttgcgccgatacaagcggcggcgaaaagttaaaaagaa

caatcaattaaataagaaccatcagtcctcgtattttgtcttctgtttgc

aagcaatgcaccaaacacaagatcaactaagaacgcatctacaa**atg**ttac (Start Codon)

gtaacacttttactagagctggtggactatcacgtatta↓catccgtaaga (Cox5a Up)

ttcgctcaaacacatgctctttccaacgctgctgtaatggatctgcaatc

cagatgggagaacatgccctccactgagcagcaggatattgtcagtaagt

tgagtgaacgtcaaaaattaccatgggcacagcttactgagcctgaaaag (Cox5a Reverse)

caagctgtgtggtacatttcttacggagaatggggcccaagaagacctgt

attgaataagggtgattccagttttattgccaaaggtgttgctgcaggcc

tactattttcagtgggactttttgctgtcgtcaggatggcgggtggccaa

gacgcaaagaccatgaataaggagtggcagctaaagagtgacgaatattt

gaagtcgaagaatgctaat↓ccttggggtggttattctcaggtccaatcta (Cox5a Down)

**aatga**acaggccagaaattttgaaaggaaatcaataggggttaacgattg (Stop Codon)

tcatggtttttcagctagtctgtgacctgtacgaaagtgaatatcttat

tacattataagtgtatccatgggcatccgcccaatacaatgccaacatat - 3'

FIG.3

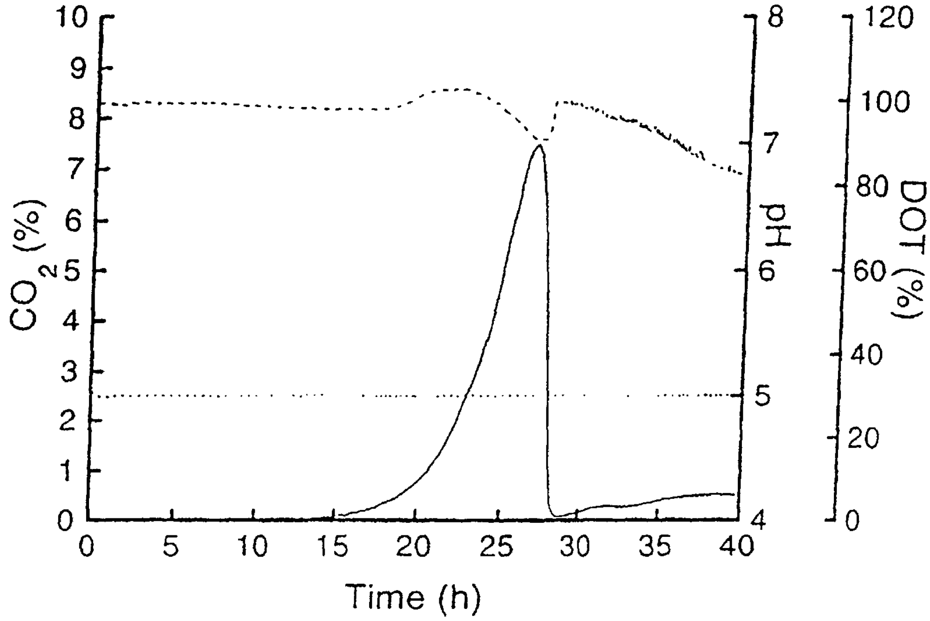
10
9
8
7
6
5
4
3
2
1
0
$CO_2$ (%)
0
5
10
15
20
25
30
35
40
Time (h)
8
7
6
5
4
pH
120
100
80
60
40
20
0
DOT (%)

FIG.4

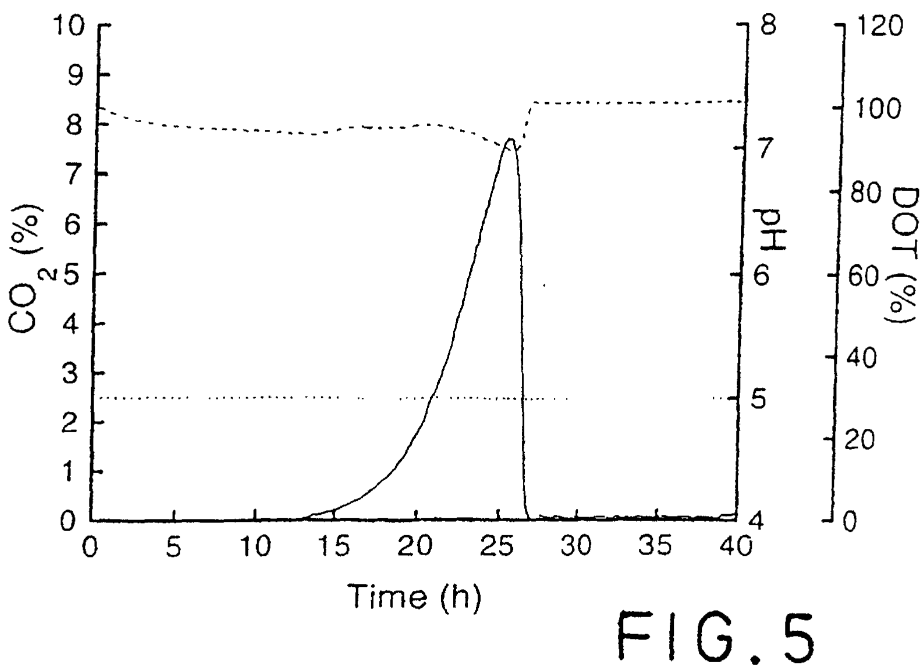
10
9
8
7
6
5
4
3
2
1
0
$CO_2$ (%)
0
5
10
15
20
25
30
35
40
Time (h)
8
7
6
5
4
pH
120
100
80
60
40
20
0
DOT (%)

FIG.5

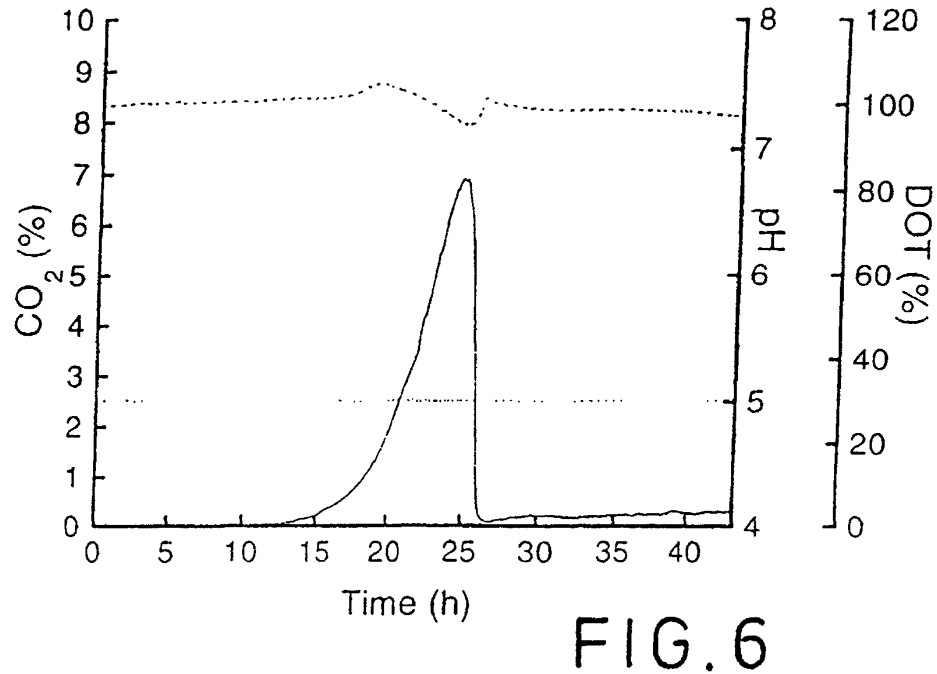
10
9
8
7
6
5
4
3
2
1
0
$CO_2$ (%)
0 5 10 15 20 25 30 35 40
Time (h)
8
7
6
5
4
pH
120
100
80
60
40
20
0
DOT (%)

FIG.6

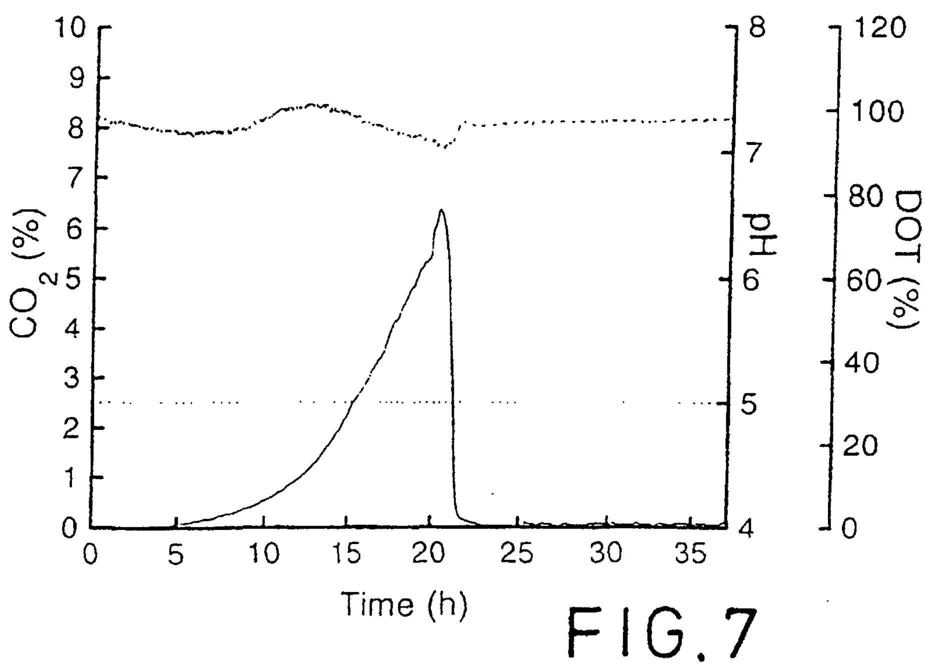
10
9
8
7
6
5
4
3
2
1
0
$CO_2$ (%)
0 5 10 15 20 25 30 35
Time (h)
8
7
6
5
4
pH
120
100
80
60
40
20
0
DOT (%)

FIG.7

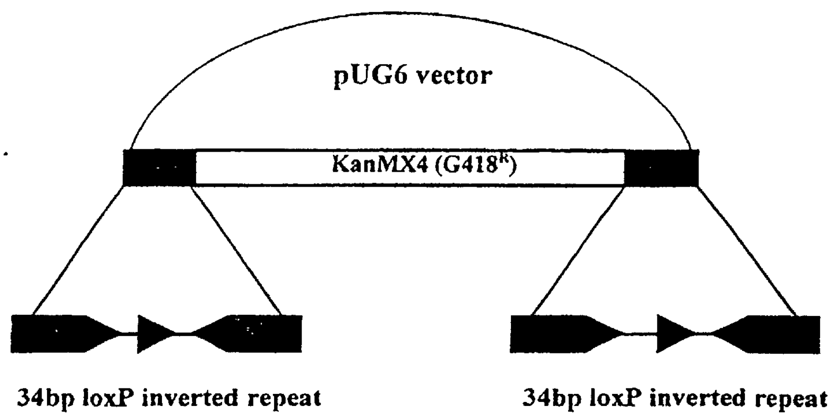
pUG6 vector
KanMX4 (G418R)
34bp loxP inverted repeat
34bp loxP inverted repeat

FIG. 8

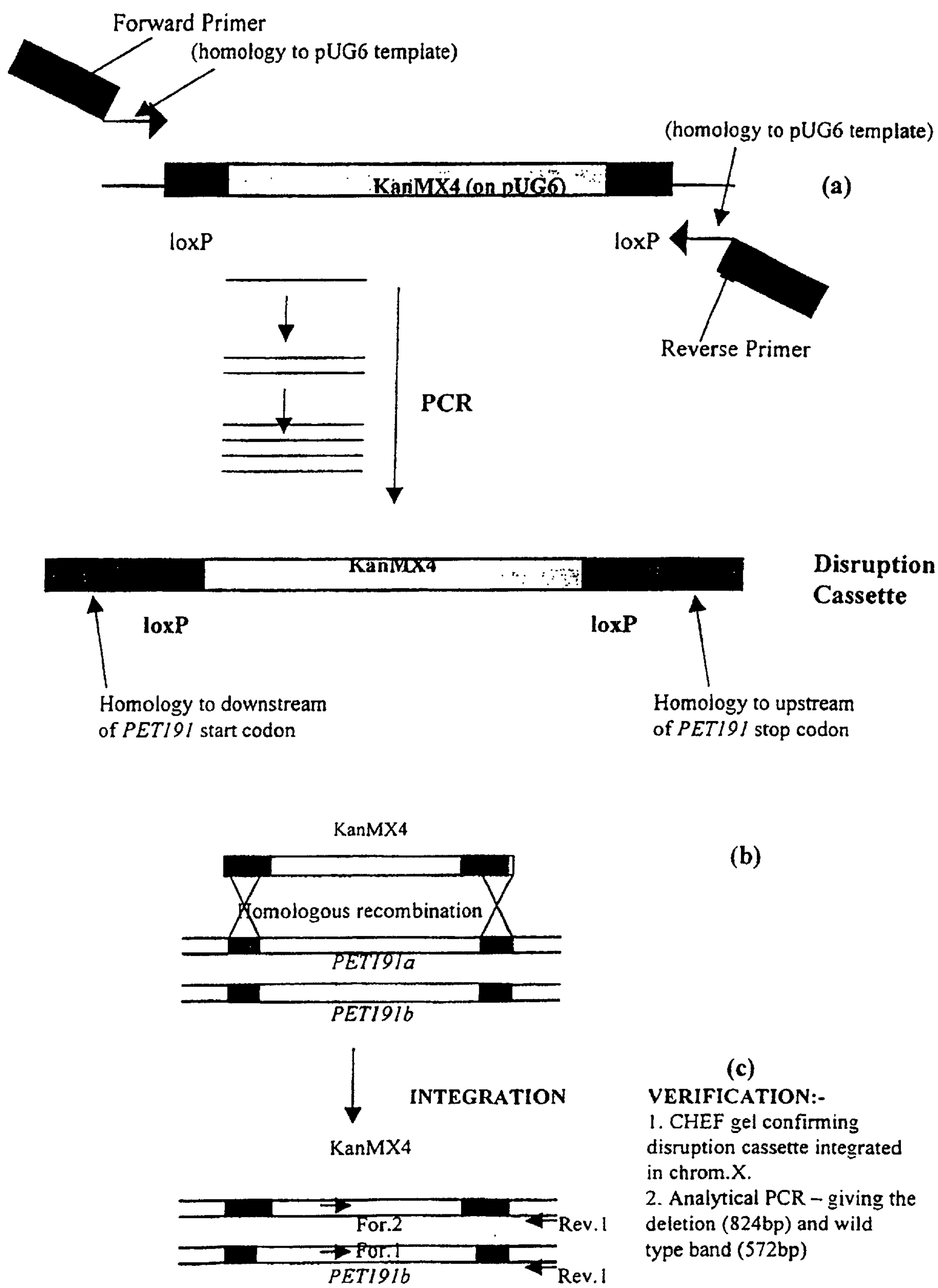
Forward Primer
(homology to pUG6 template)
(homology to pUG6 template)
KanMX4 (on pUG6)
(a)
loxP
loxP
Reverse Primer
PCR
KanMX4
Disruption Cassette
loxP
loxP
Homology to downstream of PET191 start codon
Homology to upstream of PET191 stop codon
KanMX4
(b)
Homologous recombination
PET191a
PET191b
INTEGRATION
KanMX4
For.2
Rev.1
For.1
PET191b
Rev.1
(c)
VERIFICATION:-
1. CHEF gel confirming disruption cassette integrated in chrom.X.
2. Analytical PCR – giving the deletion (824bp) and wild type band (572bp)

FIG.9

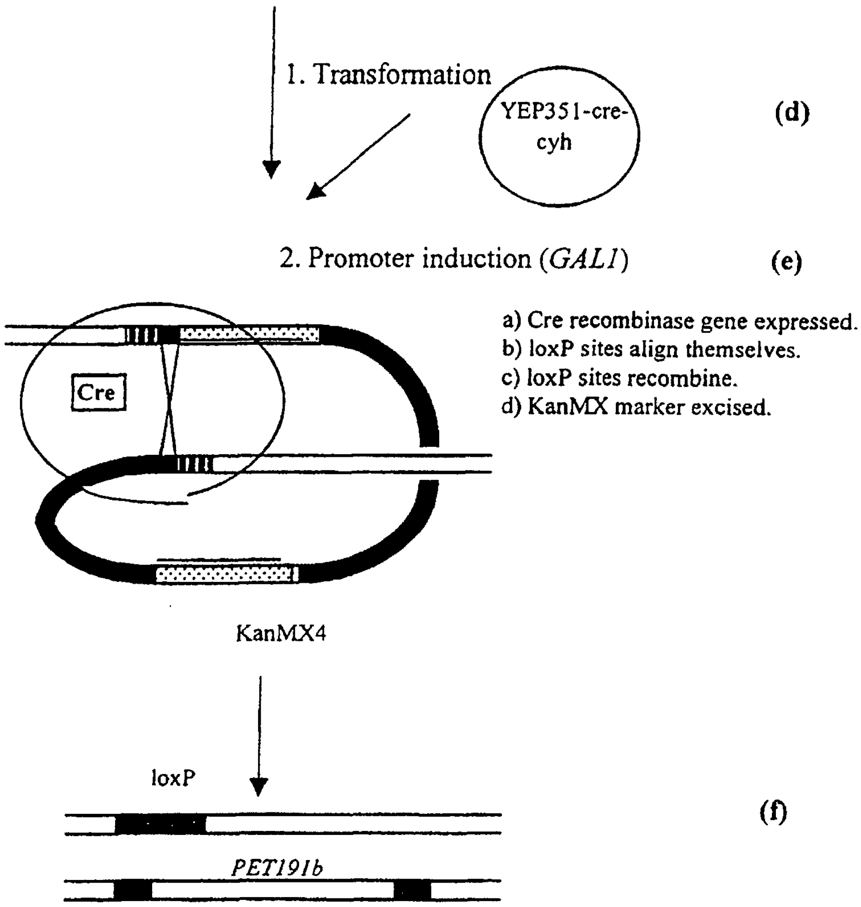


FIG. 9 continued

(a)

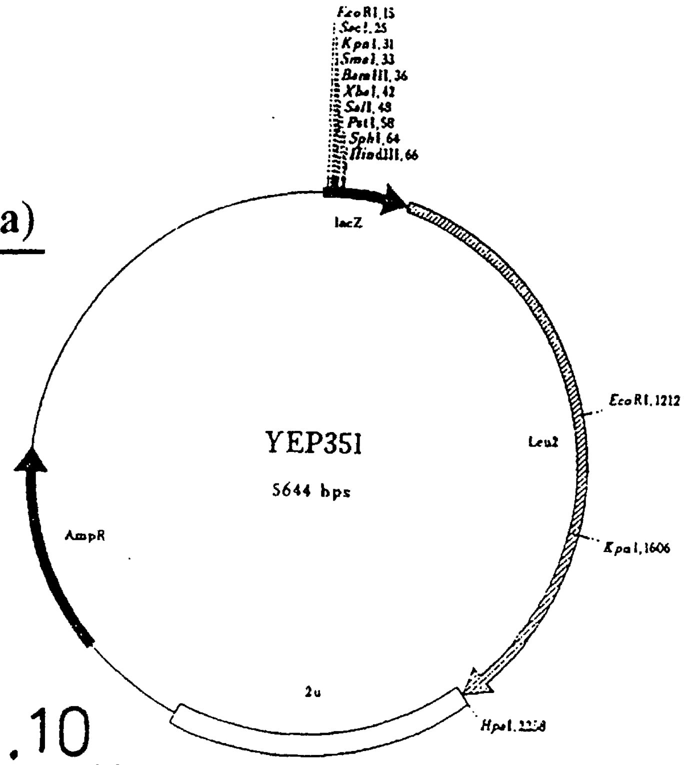


FIG.10

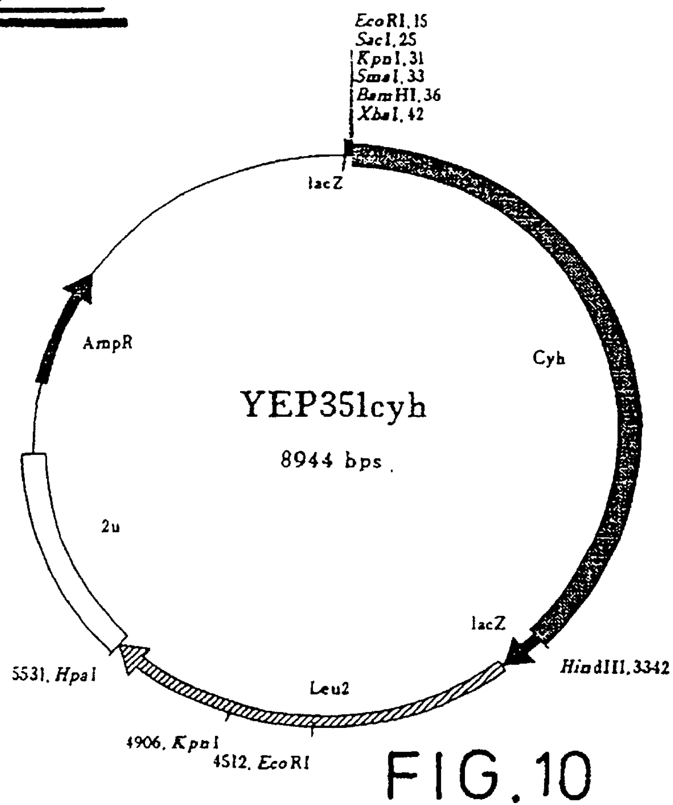


(b)

FIG.10

(c)

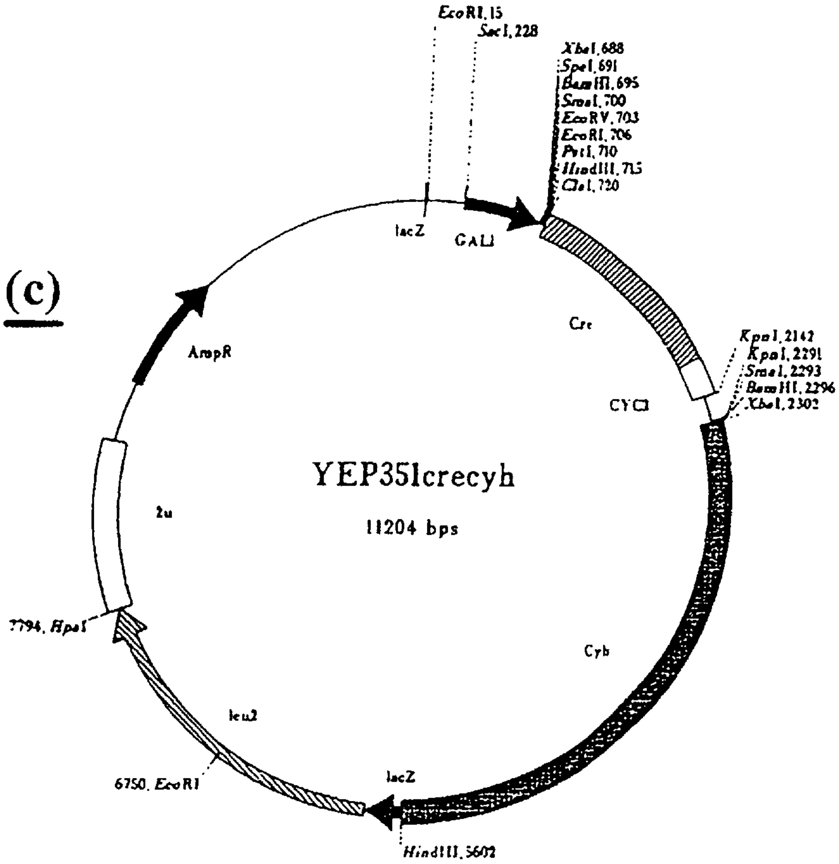
EcoRI, 15
SacI, 228
XbaI, 688
SpeI, 691
BamHI, 695
SmaI, 700
EcoRV, 703
EcoRI, 706
PstI, 710
HindIII, 715
ClaI, 720
lacZ
GAL1
Cre
KpnI, 2142
KpnI, 2291
SmaI, 2293
BamHI, 2296
XbaI, 2302
CYC1
AmpR
YEP351crecyh
11204 bps
2u
7794, HpaI
Cyb
leu2
6750, EcoRI
lacZ
HindIII, 5602

FIG. 10

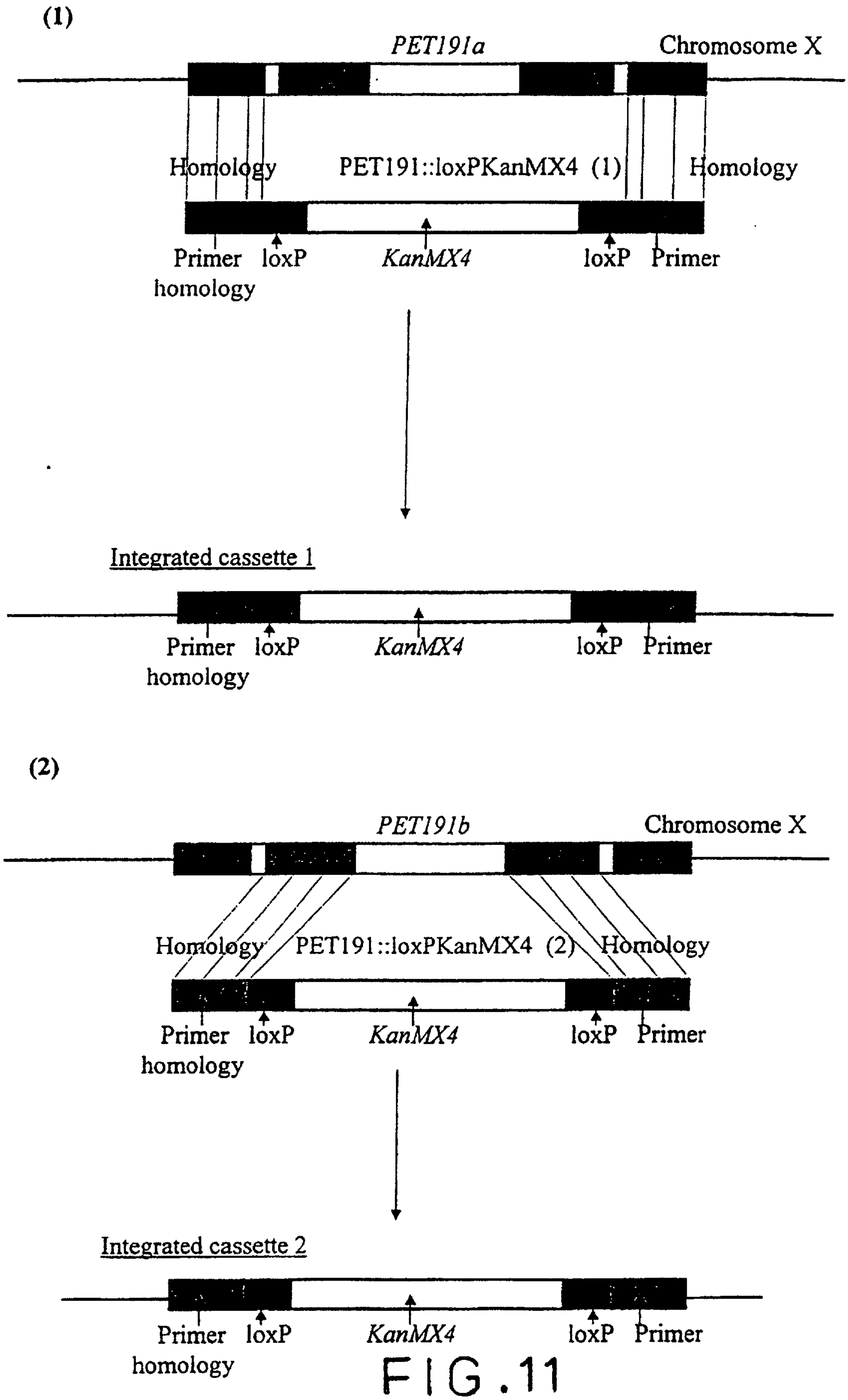
(1)
PET191a
Chromosome X
Homology
PET191::loxPKanMX4 (1)
Homology
Primer homology
loxP
KanMX4
loxP
Primer
Integrated cassette 1
Primer homology
loxP
KanMX4
loxP
Primer
(2)
PET191b
Chromosome X
Homology
PET191::loxPKanMX4 (2)
Homology
Primer homology
loxP
KanMX4
loxP
Primer
Integrated cassette 2
Primer homology
loxP
KanMX4
loxP
Primer

FIG.11

SEQUENCE LISTING

(1) GENERAL INFORMATION:

(i) APPLICANT:

(A) NAME: SACHETPACK LIMITED
(B) STREET: 6 PIENTON WAY, NORTH CHESHIRE TRADING ESTATE
(C) CITY: PRENTON
(E) COUNTRY: UNITED KINGDOM
(F) POSTAL CODE (ZIP): L43 3DU

(ii) TITLE OF INVENTION: ETHANOL PRODUCTION

(iii) NUMBER OF SEQUENCES: 19

(iv) COMPUTER READABLE FORM:

(A) MEDIUM TYPE: Floppy disk
(B) COMPUTER: IBM PC compatible
(C) OPERATING SYSTEM: PC-DOS/MS-DOS
(D) SOFTWARE: PatentIn Release #1.0, Version #1.30 (EPO)

(2) INFORMATION FOR SEQ ID NO: 1:

(i) SEQUENCE CHARACTERISTICS:

(A) LENGTH: 327 base pairs
(B) TYPE: nucleic acid
(C) STRANDEDNESS: both
(D) TOPOLOGY: linear

(ii) MOLECULE TYPE: DNA (genomic)

(iii) HYPOTHETICAL: NO

(xi) SEQUENCE DESCRIPTION: SEQ ID NO: 1:

```
TGCAGAGATC GCCCTGTGTC ATGATAGAGA GACATAACCC TCAAGAATGT CTTGACAATC    60
CAGAGCTGAA TAAGGATCTG CCCGAACTTT GTATTGCTCA GATGAAAGCA TTTTTGGATT   120
GTAAGCGAGG AATCGTCGAC ATGACTAAGC GGTTCACAGG TAACGCACCT CTGTCGACTG   180
GCAAGTACGA TCAACAGTAC GAAAACTTGT GCAAAGGAAA GTTTGATCCG AGGGAGGAGA   240
TGGAAAAGCT AAAACTTCTG AACAGCCAGC AGAAGGACTG ATTATTTATG AAAATTGGCC   300
TGTAAATATA TACATAAATA AATATAT                                        327
```

(2) INFORMATION FOR SEQ ID NO: 2:

(i) SEQUENCE CHARACTERISTICS:

(A) LENGTH: 1544 base pairs

(B) TYPE: nucleic acid
(C) STRANDEDNESS: both
(D) TOPOLOGY: linear

(ii) MOLECULE TYPE: DNA (genomic)

(iii) HYPOTHETICAL: NO

(xi) SEQUENCE DESCRIPTION: SEQ ID NO: 2:

```
CAGCTGAAGC TTCGTACGCT GCAGGTCGAC GGATCCCCGG GTTAATTAAG GCGCGCCAGA   60
TCTGTTTAGC TTGCCTCGTC CCCGCCGGGT CACCCGGCCA GCGACATGGA GGCCCAGAAT  120
ACCCTCCTTG ACAGTCTTGA CGTGCGCAGC TCAGGGGCAT GATGTGACTG TCGCCCGTAC  180
ATTTAGCCCA TACATCCCCA TGTATAATCA TTTGCATCCA TACATTTTGA TGGCCGCACG  240
GCGCGAAGCA AAAATTACGG CTCCTCGCTG CAGACCTGCG AGCAGGGAAA CGCTCCCCTC  300
ACAGACGCGT TGAATTGTCC CCACGCCGCG CCCCTGTAGA GAAATATAAA AGGTTAGGAT  360
TTGCCACTGA GGTTCTTCTT TCATATACTT CCTTTTAAAA TCTTGCTAGG ATACAGTTCT  420
CACATCACAT CCGAACATAA ACAACCATGG GTAAGGAAAA GACTCACGTT TCGAGGCCGC  480
GATTAAATTC CAACATGGAT GCTGATTTAT ATGGGTATAA ATGGGCTCGC GATAATGTCG  540
GGCAATCAGG TGCGACAATT TATCGATTGT ATGGGAAGCC CGATGCGCCA GAGTTGTTTC  600
TGAAACATGG CAAAGGTAGC GTTGCCAATG ATGTTACAGA TGAGATGGTC AGACTAAACT  660
GGCTGACGGA ATTTATGCCT CTTCCGACCA TCAAGCATTT TATCCGTACT CCTGATGATG  720
CATGGTTACT CACCACTGCG ATCCCCGGCA AAACAGCATT CCAGGTATTA GAAGAATATC  780
CTGATTCAGG TGAAAATATT GTTGATGCGC TGGCAGTGTT CCTGCGCCGG TTGCATTCGA  840
TTCCTGTTTG TAATTGTCCT TTTAACAGCG ATCGCGTATT TCGTCTCGCT CAGGCGCAAT  900
CACGAATGAA TAACGGTTTG GTTGATGCGA GTGATTTTGA TGACGAGCGT AATGGCTGGC  960
CTGTTGAACA AGTCTGGAAA GAAATGCATA AGCTTTTGCC ATTCTCACCG GATTCAGTCG 1020
TCACTCATGG TGATTTCTCA CTTGATAACC TTATTTTTGA CGAGGGGAAA TTAATAGGTT 1080
GTATTGATGT TGGACGAGTC GGAATCGCAG ACCGATACCA GGATCTTGCC ATCCTATGGA 1140
ACTGCCTCGG TGAGTTTTCT CCTTCATTAC AGAAACGGCT TTTTCAAAAA TATGGTATTG 1200
ATAATCCTGA TATGAATAAA TTGCAGTTTC ATTTGATGCT CGATGAGTTT TTCTAATCAG 1260
TACTGACAAT AAAAAGATTC TTGTTTTCAA GAACTTGTCA TTTGTATAGT TTTTTTATAT 1320
TGTAGTTGTT CTATTTTAAT CAAATGTTAG CGTGATTTAT ATTTTTTTTC GCCTCGACAT 1380
CATCTGCCCA GATGCGAAGT TAAGTGCGCA GAAAGTAATA TCATGCGTCA ATCGTATGTG 1440
AATGCTGGTC GCTATACTGC TGTCGATTCG ATACTAACGC CGCCATCCAG TGTCGAAAAC 1500
```

GAGCTCGAAT TCATCGATGA TATCAGATCC ACTAGTGGCC TATG 1544

(2) INFORMATION FOR SEQ ID NO: 3:

(i) SEQUENCE CHARACTERISTICS:

(A) LENGTH: 1598 base pairs
(B) TYPE: nucleic acid
(C) STRANDEDNESS: both
(D) TOPOLOGY: linear

(ii) MOLECULE TYPE: DNA (genomic)

(iii) HYPOTHETICAL: NO

(xi) SEQUENCE DESCRIPTION: SEQ ID NO: 3:

```
GAGCTGAAGC TTCGTACGCT GCAGGTCGAC AACCCTTAAT ATAACTTCGT ATAATGTATG     60
CTATACGAAG TTATTAGGTC TAGATTTAGC TTGCCTCGTC CCCGCCGGGT CACCCGGCCA    120
GCGACATGGA GGCCCAGAAT ACCCTCCTTG ACAGTCTTGA CGTGCGCAGC TCAGGGGCAT    180
GATGTGACTG TCGCCCGTAC ATTTAGCCCA TACATCCCCA TGTATAATCA TTTGCATCCA    240
TACATTTTGA TGGCCGCACG GCGCGAAGCA AAAATTACGG CTCCTCGCTG CAGACCTGCG    300
AGCAGGGAAA CGCTCCCCTC ACAGACGCGT TGAATTGTCC CCACGCCGCG CCCCTGTAGA    360
GAAATATAAA AGGTTAGGAT TTGCCACTGA GGTTCTTCTT TCATATACTT CCTTTTAAAA    420
TCTTGCTAGG ATACAGTTCT CACATCACAT CCGAACATAA ACAACCATGG GTAAGGAAAA    480
AATTCACGTT TCGAGGCCGC GATTAAATTC CAACATGGAT GCTGATTTAT ATGGGTATAA    540
ATGGGCTCGC GATAATGTCG GGCAATCAGG TGCGACAATC TATCGATTGT ATGGGAAGCC    600
TGATGCGCCA GAGTTGTTTC TGAAACATGG CAAAGGTAGC GTTGCCAATG ATGTTACAGA    660
TGAGATGGTC AGACTAAACT GGCTGACGGA ATTTATGCCT CTTCCGACCA TCAAGCATTT    720
TATCCGTACT CCTGATGATG CATGGTTACT CACCACTGCG ATCCCCGGCA AAACAGCATT    780
CCAGGTATTA GAAGAATATC CTGATTCAGG TGAAAATATT GTTGATGCGC TGGCAGTGTT    840
CCTGCGCCGG TTGCATTCGA TTCCTGTTTG TAATTGTCCT TTTAACAGCG ATCGCGTATT    900
TCGTCTCGCT CAGGCGCAAT CACGAATGAA TAACGGTTTG GTTGATGCGA GTGATTTTGA    960
TGACGAGCGT AATGGCTGGC CTGTTGAACA AGTCTGGAAA GAAATGCATA AGCTTTTGCC   1020
ATTCTCACCG GATTCAGTCG TCACTCATGG TGATTTCTCA CTTGATAACC TTATTTTTGA   1080
CGAGGGGAAA TTAATAGGTT GTATTGATGT TGGACGAGTC GGAATCGCAG ACCGATACCA   1140
GGATCTTGCC ATCCTATGGA ACTGCCTCGG TGAGTTTTCT CCTTCATTAC AGAAACGGCT   1200
```

TTTTCAAAAA TATGGTATTG ATAATCCTGA TATGAATAAA TTGCAGTTTC ATTTGATGCT 1260

CGATGAGTTT TTCTAATCAG TACTGACAAT AAAAAGATTC TTGTTTTCAA GAACTTGTCA 1320

TTTGTATAGT TTTTTTATAT TGTAGTTGTT CTATTTTAAT CAAATGTTAG CGTGATTTAT 1380

ATTTTTTTTC GCCTCGACAT CATCTGCCCA GATGCGAAGT TAAGTGCGCA GAAAGTAATA 1440

TCATGCGTCA ATCGTATGTG AATGCTGGTC GCTATACTGC TGTCGATTCG ATACTAACGC 1500

CGCCATCCAG TGTCGAAAAC GAGAACCCTT AATATAACTT CGTATAATGT ATGCTATACG 1560

AAGTTATTAG GTGATATCAG ATCCACTAGT GGCCTATG 1598

(2) INFORMATION FOR SEQ ID NO: 4:

(i) SEQUENCE CHARACTERISTICS:

(A) LENGTH: 237 base pairs
(B) TYPE: nucleic acid
(C) STRANDEDNESS: both
(D) TOPOLOGY: linear

(ii) MOLECULE TYPE: DNA (genomic)

(iii) HYPOTHETICAL: NO

(xi) SEQUENCE DESCRIPTION: SEQ ID NO: 4:

ATGTCTTGAC AATCCAGAGC TGAATAAGGA TCTGCCCGAA CTTTGTATTG CTCAGATGAA 60

AGCATTTTTG GATTGTAAGC GAGGAATCGT CGACATGACT AAGCGGTTCA CAGGTAACGC 120

ACCTCTGTCG ACTGGCAAGT ACGATCAACA GTACGAAAAC TTGTGCAAAG GAAAGTTTGA 180

TCCGAGGGAG GAGATGGAAA AGCTAAAACT TCTGAACACC CAGCAGAAGG ACTGATT 237

(2) INFORMATION FOR SEQ ID NO: 5:

(i) SEQUENCE CHARACTERISTICS:

(A) LENGTH: 380 base pairs
(B) TYPE: nucleic acid
(C) STRANDEDNESS: both
(D) TOPOLOGY: linear

(ii) MOLECULE TYPE: DNA (genomic)

(iii) HYPOTHETICAL: NO

(xi) SEQUENCE DESCRIPTION: SEQ ID NO: 5:

CATCCGTAAG ATTCGCTCAA ACACATGCTC TTTCCAACGC TGCTGTAATG GATCTGCAAT 60

CCAGATGGGA GAACATGCCC TCCACTGAGC AGCAGGATAT TGTCAGTAAG TTGAGTGAAC 120

```
GTCAAAAATT ACCATGGGCA CAGCTTACTG AGCCTGAAAA GCAAGCTGTG TGGTACATTT      180
CTTACGGAGA ATGGGGCCCA AGAAGACCTG TATTGAATAA GGGTGATTCC AGTTTTATTG      240
CCAAAGGTGT TGCTGCAGGC CTACTATTTT CAGTGGGACT TTTTGCTGTC GTCAGGATGG      300
CGGGTGGCCA AGACGCAAAG ACCATGAATA AGGAGTGGCA GCTAAAGAGT GACGAATATT      360
TGAAGTCGAA GAATGCTAAT                                                  380
```

(2) INFORMATION FOR SEQ ID NO: 6:

(i) SEQUENCE CHARACTERISTICS:

(A) LENGTH: 3938 base pairs
(B) TYPE: nucleic acid
(C) STRANDEDNESS: both
(D) TOPOLOGY: circular

(ii) MOLECULE TYPE: DNA (genomic)

(iii) HYPOTHETICAL: NO

(xi) SEQUENCE DESCRIPTION: SEQ ID NO: 6:

```
GAACGCGGCC GCCAGCTGAA GCTTCGTACG CTGCAGGTCG ACGGATCCCC GGGTTAATTA       60
AGGCGCGCCA GATCTGTTTA GCTTGCCTCG TCCCCGCCGG GTCACCCGGC CAGCGACATG      120
GAGGCCCAGA ATACCCTCCT TGACAGTCTT GACGTGCGCA GCTCAGGGGC ATGATGTGAC      180
TGTCGCCCGT ACATTTAGCC CATACATCCC CATGTATAAT CATTTGCATC CATACATTTT      240
GATGGCCGCA CGGCGCGAAG CAAAAATTAC GGCTCCTCGC TGCAGACCTG CGAGCAGGGA      300
AACGCTCCCC TCACAGACGC GTTGAATTGT CCCCACGCCG CGCCCCTGTA GAGAAATATA      360
AAAGGTTAGG ATTTGCCACT GAGGTTCTTC TTTCATATAC TTCCTTTTAA AATCTTGCTA      420
GGATACAGTT CTCACATCAC ATCCGAACAT AAACAACCAT GGGTAAGGAA AAGACTCACG      480
TTTCGAGGCC GCGATTAAAT TCCAACATGG ATGCTGATTT ATATGGGTAT AAATGGGCTC      540
GCGATAATGT CGGGCAATCA GGTGCGACAA TCTATCGATT GTATGGGAAG CCCGATGCGC      600
CAGAGTTGTT TCTGAAACAT GGCAAAGGTA GCGTTGCCAA TGATGTTACA GATGAGATGG      660
TCAGACTAAA CTGGCTGACG GAATTTATGC CTCTTCCGAC CATCAAGCAT TTTATCCGTA      720
CTCCTGATGA TGCATGGTTA CTCACCACTG CGATCCCCGG CAAAACAGCA TTCCAGGTAT      780
TAGAAGAATA TCCTGATTCA GGTGAAAATA TTGTTGATGC GCTGGCAGTG TTCCTGCGCC      840
GGTTGCATTC GATTCCTGTT TGTAATTGTC CTTTTAACAG CGATCGCGTA TTTCGTCTCG      900
CTCAGGCGCA ATCACGAATG AATAACGGTT TGGTTGATGC GAGTGATTTT GATGACGAGC      960
```

GTAATGGCTG GCCTGTTGAA CAAGTCTGGA AAGAAATGCA TAAGCTTTTG CCATTCTCAC 1020

CGGATTCAGT CGTCACTCAT GGTGATTTCT CACTTGATAA CCTTATTTTT GACGAGGGGA 1080

AATTAATAGG TTGTATTGAT GTTGGACGAG TCGGAATCGC AGACCGATAC CAGGATCTTG 1140

CCATCCTATG GAACTGCCTC GGTGAGTTTT CTCCTTCATT ACAGAAACGG CTTTTTCAAA 1200

AATATGGTAT TGATAATCCT GATATGAATA AATTGCAGTT TCATTTGATG CTCGATGAGT 1260

TTTTCTAATC AGTACTGACA ATAAAAAGAT TCTTGTTTTC AAGAACTTGT CATTTGTATA 1320

GTTTTTTTAT ATTGTAGTTG TTCTATTTTA ATCAAATGTT AGCGTGATTT ATATTTTTTT 1380

TCGCCTCGAC ATCATCTGCC CAGATGCGAA GTTAAGTGCG CAGAAAGTAA TATCATGCGT 1440

CAATCGTATG TGAATGCTGG TCGCTATACT GCTGTCGATT CGATACTAAC GCCGCCATCC 1500

AGTTTAAACG AGCTCGAATT CATCGATGAT ATCAGATCCA CTAGTGGCCT ATGCGGCCGC 1560

GGATCTGCCG GTCTCCCTAT AGTGAGTCGT ATTAATTTCG ATAAGCCAGG TTAACCTGCA 1620

TTAATGAATC GGCCAACGCG CGGGGAGAGG CGGTTTGCGT ATTGGGCGCT CTTCCGCTTC 1680

CTCGCTCACT GACTCGCTGC GCTCGGTCGT TCGGCTGCGG CGAGCGGTAT CAGCTCACTC 1740

AAAGGCGGTA ATACGGTTAT CCACAGAATC AGGGGATAAC GCAGGAAAGA ACATGTGAGC 1800

AAAAGGCCAG CAAAAGGCCA GGAACCGTAA AAAGGCCGCG TTGCTGGCGT TTTTCCATAG 1860

GCTCCGCCCC CCTGACGAGC ATCACAAAAA TCGACGCTCA AGTCAGAGGT GGCGAAACCC 1920

GACAGGACTA TAAAGATACC AGGCGTTTCC CCCTGGAAGC TCCCTCGTGC GCTCTCCTGT 1980

TCCGACCCTG CCGCTTACCG GATACCTGTC CGCCTTTCTC CCTTCGGGAA GCGTGGCGCT 2040

TTCTCAATGC TCACGCTGTA GGTATCTCAG TTCGGTGTAG GTCGTTCGCT CCAAGCTGGG 2100

CTGTGTGCAC GAACCCCCCG TTCAGCCCGA CCGCTGCGCC TTATCCGGTA ACTATCGTCT 2160

TGAGTCCAAC CCGGTAAGAC ACGACTTATC GCCACTGGCA GCAGCCACTG GTAACAGGAT 2220

TAGCAGAGCG AGGTATGTAG GCGGTGCTAC AGAGTTCTTG AAGTGGTGGC CTAACTACGG 2280

CTACACTAGA AGGACAGTAT TTGGTATCTG CGCTCTGCTG AAGCCAGTTA CCTTCGGAAA 2340

AAGAGTTGGT AGCTCTTGAT CCGGCAAACA AACCACCGCT GGTAGCGGTG GTTTTTTTGT 2400

TTGCAAGCAG CAGATTACGC GCAGAAAAAA AGGATCTCAA GAAGATCCTT TGATCTTTTC 2460

TACGGGGTCT GACGCTCAGT GGAACGAAAA CTCACGTTAA GGGATTTTGG TCATGAGATT 2520

ATCAAAAAGG ATCTTCACCT AGATCCTTTT AAATTAAAAA TGAAGTTTTA AATCAATCTA 2580

AAGTATATAT GAGTAAACTT GGTCTGACAG TTACCAATGC TTAATCAGTG AGGCACCTAT 2640

CTCAGCGATC TGTCTATTTC GTTCATCCAT AGTTGCCTGA CTCCCCGTCG TGTAGATAAC 2700

TACGATACGG GAGGGCTTAC CATCTGGCCC CAGTGCTGCA ATGATACCGC GAGACCCACG 2760

```
CTCACCGGCT CCAGATTTAT CAGCAATAAA CCAGCCAGCC GGAAGGGCCG AGCGCAGAAG    2820
TGGTCCTGCA ACTTTATCCG CCTCCATCCA GTCTATTAAT TGTTGCCGGG AAGCTAGAGT    2880
AAGTAGTTCG CCAGTTAATA GTTTGCGCAA CGTTGTTGCC ATTGCTACAG GCATCGTGGT    2940
GTCACGCTCG TCGTTTGGTA TGGCTTCATT CAGCTCCGGT TCCCAACGAT CAAGGCGAGT    3000
TACATGATCC CCCATGTTGT GCAAAAAAGC GGTTAGCTCC TTCGGTCCTC CGATCGTTGT    3060
CAGAAGTAAG TTGGCCGCAG TGTTATCACT CATGGTTATG GCAGCACTGC ATAATTCTCT    3120
TACTGTCATG CCATCCGTAA GATGCTTTTC TGTGACTGGT GAGTACTCAA CCAAGTCATT    3180
CTGAGAATAG TGTATGCGGC GACCGAGTTG CTCTTGCCCG GCGTCAATAC GGGATAATAC    3240
CGCGCCACAT AGCAGAACTT TAAAAGTGCT CATCATTGGA AAACGTTCTT CGGGGCGAAA    3300
ACTCTCAAGG ATCTTACCGC TGTTGAGATC CAGTTCGATG TAACCCACTC GTGCACCCAA    3360
CTGATCTTCA GCATCTTTTA CTTTCACCAG CGTTTCTGGG TGAGCAAAAA CAGGAAGGCA    3420
AAATGCCGCA AAAAAGGGAA TAAGGGCGAC ACGGAAATGT TGAATACTCA TACTCTTCCT    3480
TTTTCAATAT TATTGAAGCA TTTATCAGGG TTATTGTCTC ATGAGCGGAT ACATATTTGA    3540
ATGTATTTAG AAAAATAAAC AAATAGGGGT TCCGCGCACA TTTCCCCGAA AAGTGCCACC    3600
TGACGTCTAA GAAACCATTA TTATCATGAC ATTAACCTAT AAAAATAGGC GTATCACGAG    3660
GCCCTTTCGT CTCGCGCGTT TCGGTGATGA CGGTGAAAAC CTCTGACACA TGCAGCTCCC    3720
GGAGACGGTC ACAGCTTGTC TGTAAGCGGA TGCCGGGAGC AGACAAGCCC GTCAGGGCGC    3780
GTCAGCGGGT GTTGGCGGGT GTCGGGGCTG GCTTAACTAT GCGGCATCAG AGCAGATTGT    3840
ACTGAGAGTG CACCATATGG ACATATTGTC GTTAGAACGC GGCTACAATT AATACATAAC    3900
CTTATGTATC ATACACATAC GATTTAGGTG ACACTATA                            3938
```

(2) INFORMATION FOR SEQ ID NO: 7:

(i) SEQUENCE CHARACTERISTICS:

(A) LENGTH: 54 base pairs
(B) TYPE: nucleic acid
(C) STRANDEDNESS: single
(D) TOPOLOGY: linear

(ii) MOLECULE TYPE: DNA (genomic)

(iii) HYPOTHETICAL: NO

(xi) SEQUENCE DESCRIPTION: SEQ ID NO: 7:

```
ATGTAAAGAT CAGAAGAAGG CTGTCGCTAT ATGTTCAGCT GAAGCTTCGT ACGC    54
```

(2) INFORMATION FOR SEQ ID NO: 8:

(i) SEQUENCE CHARACTERISTICS:

(A) LENGTH: 56 base pairs
(B) TYPE: nucleic acid
(C) STRANDEDNESS: single
(D) TOPOLOGY: linear

(ii) MOLECULE TYPE: DNA (genomic)

(iii) HYPOTHETICAL: NO

(xi) SEQUENCE DESCRIPTION: SEQ ID NO: 8:

GATTACTGGA CAAACCGAAT AAAAGAGCTT GACGCCATAG GCCACTAGTG GATCTG 56

(2) INFORMATION FOR SEQ ID NO:

(i) SEQUENCE CHARACTERISTICS:

(A) LENGTH: 17 base pairs
(B) TYPE: nucleic acid
(C) STRANDEDNESS: single
(D) TOPOLOGY: linear

(ii) MOLECULE TYPE: DNA (genomic)

(iii) HYPOTHETICAL: NO

(xi) SEQUENCE DESCRIPTION: SEG ID NO: 9:

GCACCTCTGT CGACTGG 17

(2) INFORMATION FOR SEQ ID NO:1 :

(i) SEQUENCE CHARACTERISTICS:

(A) LENGTH: 19 base pairs
(B) TYPE: nucleic acid
(C) STRANDEDNESS: single
(D) TOPOLOGY: linear

(ii) MOLECULE TYPE: DNA (genomic)

(iii) HYPOTHETICAL: NO

(xi) SEQUENCE DESCRIPTION: SEQ ID NO: 10:

GAAAGCACGT TAACTCCCA 19

(2) INFORMATION FOR SEQ ID NO: 11:

(i) SEQUENCE CHARACTERISTICS:

(A) LENGTH: 54 base pairs
(B) TYPE: nucleic acid
(C) STRANDEDNESS: single
(D) TOPOLOGY: linear

(ii) MOLECULE TYPE: DNA (genomic)

(iii) HYPOTHETICAL: NO

(xi) SEQUENCE DESCRIPTION: SEQ ID NO: 11:

CACTTTTACT AGAGCTGGTG GACTATCACG TATTACAGCT GAAGCTTCGT ACGC 54

(2) INFORMATION FOR SEQ ID NO: 12:

(i) SEQUENCE CHARACTERISTICS:

(A) LENGTH: 57 base pairs
(B) TYPE: nucleic acid
(C) STRANDEDNESS: single
(D) TOPOLOGY: linear

(ii) MOLECULE TYPE: DNA (genomic)

(iii) HYPOTHETICAL: NO

(xi) SEQUENCE DESCRIPTION: SEQ ID NO: 12:

TCATTTAGAT TGGACCTGAG AATAACCACC CCAAGGCATA GGCCACTACT GGATCTG 57

(2) INFORMATION FOR SEQ ID NO: 13:

(i) SEQUENCE CHARACTERISTICS:

(A) LEHGTH: 16 base pairs
(B) TYPE: nucleic acid
(C) STRANDEDNESS: single
(D) TOPOLOGY: linear

(ii) MOLECULE TYPE: DNA (genomic)

(iii) HYPOTHETICAL: NO

(xi) SEQUENCE DESCRIPTION: SEQ ID NO: 13:

CGCCTCCCTA CGCTTC 16

(2) INFORMATION FOR SEQ ID NO: 14:

(i) SEQUENCE CHARACTERISTICS:

(A) LENGTH: 18 base pairs
(B) TYPE: nucleic acid
(C) STRANDEDNESS: single
(D) TOPOLOGY: linear

(ii) MOLECULE TYPE: DNA (genomic)

(iii) HYPOTHETICAL: NO

(xi) SEQUENCE DESCRIPTION: SEQ ID NO: 14:

GCTCAGTAAG CTGTGCCC 18

(2) INFORMATION FOR SEQ ID NO: 15:

(i) SEQUENCE CHARACTERISTICS:

(A) LENGTH: 21 base pairs
(B) TYPE: nucleic acid
(C) STRANDEDNESS: single
(D) TOPOLOGY: linear

(ii) MOLECULE TYPE: DNA (genomic)

(iii) HYPOTHETICAL: NO

(xi) SEQUENCE DESCRIPTION: SEQ ID NO: 15:

TCTCCTTCAT TACAGAAACG G 21

(2) INFORMATION FOR SEQ ID NO: 16:

(i) SEQUENCE CHARACTERISTICS:

(A) LENGTH: 21 base pairs
(B) TYPE: nucleic acid
(C) STRANDEDNESS: single
(D) TOPOLOGY: linear

(ii) MOLECULE TYPE: DNA (genomic)

(iii) HYPOTHETICAL: NO

(xi) SEQUENCE DESCRIPTION: SEQ ID NO: 16:

CCGTTTCTGT AATGAAGGAG A 21

(2) INFORMATION FOR SEQ ID NO: 17:

(i) SEQUENCE CHARACTERISTICS:

(A) LENGTH: 34 base pairs
(B) TYPE: nucleic acid
(C) STRANDEDNESS: both
(D) TOPOLOGY: linear

(ii) MOLECULE TYPE: DNA (genomic)

(iii) HYPOTHETICAL: NO

(xi) SEQUENCE DESCRIPTION: SEQ ID NO: 17:

ATAACTTCGT ATAATGTATG CTATACGAAG TTAT 34

(2) INFORMATION FOR SEQ ID NO: 18:

(i) SEQUENCE CHARACTERISTICS:

(A) LENGTH: 46 base pairs
(B) TYPE: nucleic acid
(C) STRANDEDNESS: single
(D) TOPOLOGY: linear

(ii) MOLECULE TYPE: DNA (genomic)

(iii) HYPOTHETICAL: NO

(xi) SEQUENCE DESCRIPTION: SEQ ID NO: 18:

GAGATCGCCC TGTGTCATGA TAGAGAGACA TAACCCTCAA GACAGC 46

(2) INFORMATION FOR SEQ ID NO: 19:

(i) SEQUENCE CHARACTERISTICS:

(A) LENGTH: 48 base pairs
(B) TYPE: nucleic acid
(C) STRANDEDNESS: single
(D) TOPOLOGY: linear

(ii) MOLECULE TYPE: DNA (genomic)

(iii) HYPOTHETICAL: NO

(xi) SEQUENCE DESCRIPTION: SEQ ID NO: 19:

TTTATTTATG TATATATTTA CAGGCCAATT TTCATAAATA CATAGGCC 48